# EUROPEAN PATENT SPECIFICATION

(11) **EP 2 886 126 B1**
(45) Date of publication and mention of the grant of the patent: **07.06.2017**
(21) Application number: 13199325.5
(22) Date of filing: 23.12.2013
(51) Int. Cl.: A61K 38/16, C07K 14/705, A61P 35/00

(54) **Nanoparticle conjugated to CD44 binding peptides**
An CD44-bindende Peptide konjugierte Nanopartikel
Nanoparticule conjuguée á peptides de liaison CD44

(43) Date of publication of application: 24.06.2015
(73) Proprietor: Exchange Imaging Technologies GmbH, 64287 Darmstadt (DE)
(72) Inventor: Arntz, Claudia, 49525 Lengerich (DE); Greb, Wolfgang, 40489 Düsseldorf (DE)
(74) Representative: Jostarndt Patentanwalts-AG

(56) References cited:
- WO-A1-95/04547
- WO-A1-2005/007700
- WO-A1-2015/097170
- DATABASE Geneseq [Online] 18 November 2004 (2004-11-18), "Human Elk1 phosphorylation/Elk1 kinase activation protein - SEQ ID 10.", XP002725223, retrieved from EBI accession no. GSP:ADR58907 Database accession no. ADR58907 -& WO 2004/072277 A2 (ASAHI KASEI PHARMA CORP [JP]; MATSUZAKI OSAMU [JP]; MATSUDA AKIO [JP]) 26 August 2004 (2004-08-26)
- DATABASE Geneseq [Online] 9 July 2009 (2009-07-09), "Human pancreatic cancer associated target (PCAT) protein, SEQ ID:14.", XP002725224, retrieved from EBI accession no. GSP:AWV72632 Database accession no. AWV72632 -& US 2009/138977 A1 (DOMON BRUNO [US] ET AL) 28 May 2009 (2009-05-28)
- DATABASE Geneseq [Online] 13 October 2011 (2011-10-13), "Human lung cancer target LCAT protein sequence SEQ: 1.", XP002725225, retrieved from EBI accession no. GSP:AZM18611 Database accession no. AZM18611 -& US 2011/212085 A1 (JOSELOFF ELIZABETH [US] ET AL) 1 September 2011 (2011-09-01)
- SEKI KENICHIRO ET AL: "Inhibition of liver metastasis formation by anti-CD44 variant exon 9 monoclonal antibody", INTERNATIONAL JOURNAL OF ONCOLOGY, DEMETRIOS A. SPANDIDOS ED. & PUB, GR, vol. 11, no. 6, 1 December 1997 (1997-12-01), pages 1257-1261, XP009124463, ISSN: 1019-6439
- Youhei Kimura ET AL: "CD44variant exon 9 plays an important role in colon cancer initiating cells", Oncotarget, 6 June 2013 (2013-06-06), pages 785-791, XP55120901, United States Retrieved from the Internet: URL:http://www.ncbi.nlm.nih.gov/pubmed/238 00986 [retrieved on 2014-05-30]
- HYE-YEON PARK ET AL: "Screening of Peptides Bound to Breast Cancer Stem Cell Specific Surface Marker CD44 by Phage Display", MOLECULAR BIOTECHNOLOGY ; PART B OF APPLIED BIOCHEMISTRY AND BIOTECHNOLOGY, HUMANA PRESS INC, NEW YORK, vol. 51, no. 3, 7 October 2011 (2011-10-07), pages 212-220, XP035059773, ISSN: 1559-0305, DOI: 10.1007/S12033-011-9458-7
- DATABASE Geneseq [Online] 22 July 2010 (2010-07-22), "Human cell surface adhesion molecule (CD44vRA) peptide, SEQ ID 23.", XP002729839, retrieved from EBI accession no. GSP:AYB86388 Database accession no. AYB86388 -& WO 2010/058396 A1 (YISSUM RES DEV CO [IL]; NAOR DAVID [IL]; NEDVETZKI SHLOMO [IL]; GOLAN) 27 May 2010 (2010-05-27)
- DATABASE Geneseq [Online] 23 September 1998 (1998-09-23), "Human CD44 exon v5 protein sequence.", XP002729840, retrieved from EBI accession no. GSP:AAW59423 Database accession no. AAW59423 -& DE 196 48 209 A1 (BOEHRINGER INGELHEIM INT [DE]) 28 May 1998 (1998-05-28)
- AFIFY ALAA M ET AL: "Expression of CD44S and CD44v5 is more common in stage III than in stage I serous ovarian carcinomas", APPLIED IMMUNOHISTOCHEMISTRY, LIPPINCOTT WILLIAMS AND WILKINS, PHILADELPHIA, PA, US, vol. 9, no. 4, 1 December 2001 (2001-12-01), pages 309-314, XP009178409, ISSN: 1062-3345
- "Homo sapiens cDNA FLJ44468 fis, clone UTERU2026025, moderately similar to SPLICING FACTOR, ARGININE/SERINE-RICH 2", UNIPROT , XP002738474, Retrieved from the Internet: URL:http://www.ebi.ac.uk/ena/data/view/AK1 26432&display=text [retrieved on 2003-09-09]
- FANG D ET AL: "Human derived cancer-associated target protein SEQ ID NO:673", GENESEQ,, 1 May 2012 (2012-05-01), XP002738475, [retrieved on 2012-06-21] & US 8 168 586 B1 (FANG DONG [US] ET AL) 1 May 2012 (2012-05-01)
- MUZNY D M ET AL: "SubName: Full=Sodium/potassium-transporting ATPase subunit beta-3 {ECO:0000313|Ensembl:ENSP00000418353}", UNIPROT,, 1 January 2006 (2006-01-01), XP002738476, [retrieved on 2009-11-03]
- DOMON B ET AL: "Human Na-K ATPase beta3 CAT protein SEQ ID:130", GENESEQ,, 1 September 2009 (2009-09-01), XP002738477, [retrieved on 2010-02-18] & US 7 582 441 B1 (RUBEN STEVE [US] ET AL) 1 September 2009 (2009-09-01)
- DATABASE GENESEQ [Online] 07 August 2008 'N-linked glycopeptide SEQ:101.' Retrieved from EBI, accession no. GSP:ARW93074 Database accession no. ARW93074 -& DATABASE GENESEQ [Online] 07 August 2008 'N-linked glycopeptide SEQ:100.' Retrieved from EBI, accession no. GSP:ARW93073 Database accession no. ARW93073 & WO 2008/066629 A2 (INST SYSTEMS BIOLOGY [US]; AEBERSOLD RUDOLF H [CH]; ZHANG HUI [US]; HO) 5 June 2008 (2008-06-05)
- DATABASE GENESEQ [Online] 05 November 2001 'Human brain expressed single exon probe encoded protein SEQ ID NO: 34941.' Retrieved from EBI, accession no. GSP:AAM62836 Database accession no. AAM62836 & WO 01/57275 A2 (MOLECULAR DYNAMICS INC [US]; PENN SHARRON G [US]; HANZEL DAVID K [US];) 9 August 2001 (2001-08-09)

## Description

### FIELD OF THE INVENTION

The present invention relates to nanoparticles conjugated to a protein capable of binding to the domain encoded by exon 9 of human CD44 (CD44ex9). The invention further relates to a method of production for the protein and the respective conjugated nanoparticles and the use of the conjugates of the invention for treatment and diagnosis of various diseases, and especially for cancer.

### BACKGROUND OF THE INVENTION

Extensive studies of cancer transcriptional patterns have led to the discovery of molecular targets to distinguish the malignant from the benign and the most aggressive cancers from those that are less aggressive. Cancers often overexpress a number of proteins, including certain cell surface antigens, e.g. cell surface receptors. Antibodies that bind such overexpressed cell surface antigens can facilitate detection and treatment of such cancers. A number of approaches have been utilized to generate antibodies to cancer cell surface receptors which can be used as potential diagnostics or therapeutics. Identification of overexpressed cell surface receptors and antibodies which bind them provide a route to the development of cancer therapies, especially for those cancer subtypes with poor prognosis and resistance to traditional therapies. CD44, CA19-9 and CEA are such overexpressed cell surface receptors, which are known to be relevant for tumor progression and malignancy.

CD44, a major adhesion molecule for the extracellular matrix, has been implicated in a wide variety of physiological processes, including leukocyte homing and activation, wound healing, and cell migration, as well as in tumor cell invasion and metastasis (Günthert et al., 1991; Nagano and Saya, 2004; Ponta et al., 2003).

CD44 is a single chain molecule comprising a conserved N-terminal extracellular domain, a non-conserved membrane proximal region, a variable region expressing various combinations of variant exons, a conserved transmembrane spanning domain and a conserved cytoplasmic tail. The genomic map of CD44 includes 5 constant exons at the 5' terminus and 5 constant exons in the 3' end. The mouse CD44 gene includes also 10 variant exons in the middle of the molecule designated V1-V10 resulting in a total of 20 exons. The human CD44 gene comprises only 9 of these 10 variant exons (V2-V10) thus comprising a total of 19 exons. Differential alternative splicing generates many isoforms of CD44 that express various combinations of variant exons (designated Exon Vₓ, x = 1 to 10), which are inserted in the membrane proximal domain and constitute the variable region of the molecule. These molecules are designated CD44 variants (CD44v). To date, 20 isoforms of CD44 are known.

Whereas the standard CD44 isoform (CD44s) is expressed predominantly in hematopoietic cells and normal epithelial cell subsets, the CD 44 variants with insertions in the membrane-proximal extracellular region were found to be abundant in a variety of human tumors, including colon, mammary, gastric, bladder, prostate carcinomas, and various hematopoietic neoplasms. Additional reports suggested a close correlation between expression of the variant CD44 and tumor progression and in particular the lymphatic spread of neoplastic cells.

Taken together, these observations point to an important function of CD44 variants in tumor initiation and the maintenance of cancer cells in addition to its more established functions in cell adhesion and migration.

Beside the role of CD44 in cancer, CD44 has also been suggested as a potential target in autoimmune diseases. It has been reported (Hale et al., 1992) that administration of a CD44 protein or peptide or derivative can be used for treating various autoimmune diseases.

Based on the established role of CD44 in cancer and autoimmune diseases, monoclonal antibodies directed against various variant regions of CD44 have also been generated as potential agents for diagnosis or therapy of CD44-related disorders.

Seiter et al. describe mAbs directed against metastasis-specific variants of CD44 surface protein of a rat pancreatic adenocarcinoma (Seiter et al., 1993). These antibodies are proposed for producing immunosuppression for therapeutic treatment of immunoregulatory disorders including, for example, diseases of the rheumatic type. Monoclonal antibodies reactive with CD44 which inhibit T-cell proliferation were also provided for treatment of various autoimmune diseases. Monoclonal antibodies binding to forms of CD44 containing the exon v6 peptide were also reported as being useful for diagnosing inflammatory diseases (Jalkanen et al., 1986).

The WO 91/17248 A1 relates to the use of anti-CD44v antibodies for the therapy and diagnosis of tumors. WO 95/00851 A1 relates to the use of antibodies directed against the variable exons of CD44 for diagnosis of tumors. The WO 95/04547 A1 discloses the use of anti-CD44 antibodies especially directed against epitopes within the sequence of exon v5 for immunotherapeutic and immunoscintigraphic purposes. An anti-CD44 antibody directed against exon v6 is disclosed by WO 95/33771 A1. Furthermore, the EP 0 538 754 A2 teaches the use of antibodies directed against CD44 variants for immunosuppression.

The anti-CD44 antibodies of the prior art show several disadvantages. They represent large and complex molecules which have to be generated by recombinant production methods. Hence, the production process is elaborate, costly and has to fulfill strict regulatory requirements. Furthermore, the necessity for a reduced immunogenic potential requires the generation of human or humanized antibodies.

The database entry no. ADR58907, retrieved from EBI accession no. GSP:ADR58907 (DATABASE Geneseq [Online] 18 November 2004, "Human Elk1 phosphorylation/Eik1 kinase activation protein- SEQ ID 10.") and WO 2004/072277 A2 (ASAHI KASEI PHARMA CORP [JP]; MATSUZAKI OSAMU [JP]; MATSUDA AKIO [JP]; 26 August 2004) disclose a peptide of 184 amino acids which is 100% identical to SEQ ID No. 2 of the present application in a 32 aa overlap. Notably, this database entry does not teach any binding to CD44.

The database entry. No. AWV72632 (retrieved from EBI accession no. GSP:AWV72632, DATABASE Geneseq [Online] 9 July 2009, "Human pancreatic cancer associated target (PCAT) protein, SEQ ID14.") and US 2009/138977 A1 (DOMON BRUNO [US] ET AL; 28 May 2009) disclose a protein of 198 amino acid which comprises aa 33 to 66 of SEQ ID. No. 4 of the present invention with only 1 amino acid difference. For said protein no binding to CD44 is disclosed.
The database entry No. AZM18611 (retrieved from EBI accession no. GSP: AZM18611, DATABASE Geneseq [Online] 13 October 2011, "Human lung cancer target LCAT protein sequence SEQ: 1.") and US 2011/212085 A1 (JOSELOFF ELIZABETH [US] ET AL; 1 September 2011) disclose a human lung cancer target LCAT protein of 102 amino acids compoeseing complete 62 aa long SEQ ID NO: 5 of the application. There is no disclosure of binding to CD44.

The application WO 1995/04547 A1 (Boehringer Ingelheim Int. [OE]; Kernforschungszentrum Karlsruhe [OE]; Adolf G) 16 February 1995) discloses

Kenichiro et al. ("Inhibition of liver metastasis formation by anti CD44 variant exon 9 monoclonal antibody", Int. J. Oncology, 1997, vol. 11, no. 6, pages 1257-1261) discloses the ability of a monoclonal antibody directed against CD44 variant exon 9 to inhibit liver metastasis formation. However, Kenichiro et al. is silent on peptides binding to CD44 exon 9.

Kimura et al, 2013 ("CD44variant exon 9 plays an important role in colon cancer initiating cells", Oncotarget, pages 785-791) pronounces the important role of the CD44variant exon 9 for the initiation of colon cancer but does not disclose CD4v9 binding peptides.

Park et al. ("Screening of Peptides Bound to Breast Cancer Stem Cell Specific Surface Marker CD44 by Phage Display", Molecular Biotechnology, 2011, vol. 51, no. 3, pages 212-220) relates of a phage display peptide screening against CD44 but does not disclose any peptide related to the peptides of the present invention.

Database entry No. AZV55426 (retrieved from EBI accession no. GSP: AZV55426; database Geneseq [Online] 21 June 2012, "Human derived cancer-associated target protein SEQ ID NO: 673.") and US 8 168 586 B1 (Fang Dong et al., 1 May 2012) disclose a sequence with 57 aa and having 83.3% identity to the whole sequence of SEQ ID No: 4 of the present invention. Said protein is identified as a human cancer-associated target protein. However, there is no reference of binding to CD44 exon 9.

Database entry No. C9J6S2 (UniProt Online database, 3 November 2009; "SubName: Full = Sodium/potassium-transporting ATPase subunit beta-3 {EC 0:00003131 Ensembl: ENSP00000418353};" retrieved from EBI accession no. UNIPROT:C9J6S2) and Burkhard et al. ("Initial characterization of the human central proteome", BMC Systems Biology 2011, vol. 5, no. 1, page 17) disclose a human Sodium/potassium-transporting ATPase subunit beta-3 with 89 aa and having 83.3% identity to the whole sequence of SEQ ID No: 4 of the present invention. There is no reference of binding to CD44 exon 9.

Database entry No. AXT76926 (retrieved from EBI accession no. GSP: AXT76926; Geneseq Online, 18 February 2010; "Human Na-K ATPase beta3 CAT protein SEQ ID:130.") and US 7 582 441 B1 (Ruben S et al., 1 September 2009) disclose a
(2009-09-01) disclose a human Na-K ATPase beta3 CAT protein of 57 aa and having 89.5 % identity to the whole sequence of SEQ ID No: 4 of the present invention. There is no reference of binding to CD44 exon 9.

Database entry No. ARW93074 (retrieved from EBI accession No. GSP: ARW9307; Geneseq Online 7 August 2008, "N-linked glycopeptide SEQ: 101."), database entry No. ARW9307 (retrieved from EBI accession No. GSP: ARW93073, database Geneseq Online, 7 August 2008, "N-linked glycopeptide SEQ: 100.") and WO 2008/066629 A2 (Inst Systems Biology, Aebersold R, Zhang H, 5 June 2008) disclose sequence ID No: 100 of 25 amino acids and sequence ID No: 101 of 32 amino acids both having 54.9% identity to the whole sequence of SEQ ID No: 4.

Database entry No. AAM62836 (retrieved from EBI accession No. GSP: AAM62836, database Geneseq Online, 5 November 2001, "Human brain expressed single exon probe encoded protein SEQ ID NO: 34941.") and WO 01/57275 A2 (Molecular Dynamics Inc.; Penn Sharron G; Hanzel Oavio K; 9 August 2001) disclose a protein of 63 amino acids, expressed in human brain and having an identity of 61.3% to the SEQ ID No: 5 of the present invention.

Hence, there is still the need for CD44 variant-binding molecules. The objective of the present invention thus is to provide a CD44 variant binding substance which overcomes at least one of the above mentioned disadvantages.

This problem is solved by provision of a CD44-variant binding protein according to claim 1 and a use of said binding protein for diagnosis and therapy, especially by using nanoparticles coated with said protein. Specific embodiments of the invention are subject matter of further independent or dependent claims.

### SUMMARY OF THE INVENTION

In a first aspect the invention provides nanoparticles conjugated to a protein capable of binding to a polypeptide encoded by exon 9 of human CD44 (CD44ex9), said protein comprises or consists of
a) an amino acid sequence according SEQ ID No. 1 to 5; or
b) an amino acid sequence with at least 95% identity to the amino acid sequence given in SEQ ID No. 1 to 5 as a whole,
wherein said protein has a length of 100 amino acids or less.

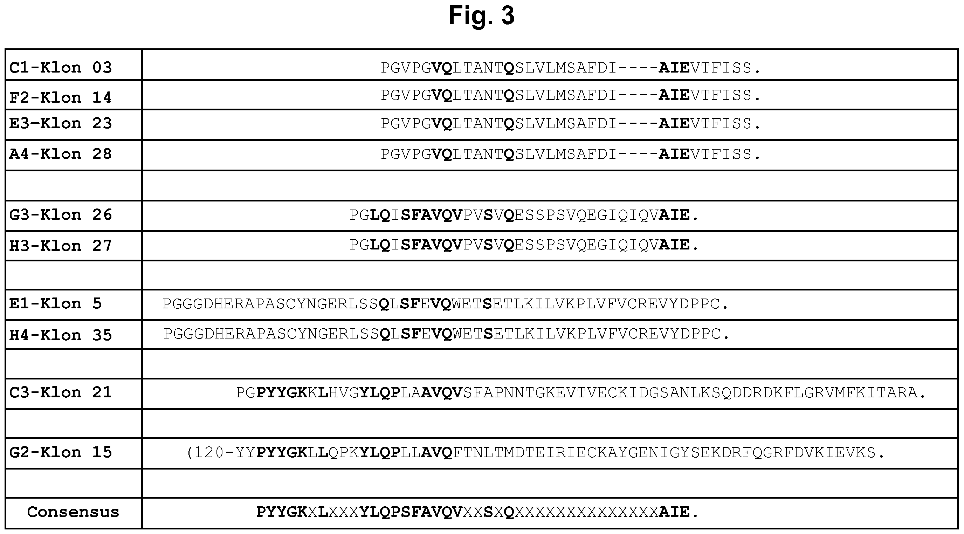

By performing a yeast-two hybrid-screening the inventors were able to identify five different peptides with a specific binding to the polypeptide encoded by exon 9 of CD44. The amino acid sequences are presented in Figure 3 and in the following table and designated as SEQ ID No. 1 to 5.

| **SEQ ID No.** | **Amino acid sequence** |
|---|---|
| 1 | PGVPGVQLTA NTQSLVLMSA FDIAIEVTFI SS |
| 2 | PGLQISFAVQ VPVSVQESSP SVQEGIQIQV AIE |
| 3 | |
| 4 | PGPYYGKKLH VGYLQPLAAV QVSFAPNNTG KEVTVECKID GSANLKSQDD RDKFLGRVMF KITARA |
| 5 | YYPYYGKLLQ PKYLQPLLAV QFTNLTMDTE IRIECKAYGE NIGYSEKDRF QGRFDVKIEV KS |

A further analysis revealed that a consensus sequence can be derived from these sequences exhibiting the following amino acid sequence:
PYYGKXLX₃YLQPSFAVQVX₂SXQX₁₀₋₁₄AIE.

These consensus sequences are designated as SEQ ID No. 6 to 10 as depicted in the following table whereby these five sequences differ in the length of the arbitrary sequence X₁₀₋₁₄. Please note that "X" is defined as an arbitrary amino acid.

| **SEQ ID No.** | **Amino acid sequence** |
|---|---|
| 6 | PYYGKXLXXX YLQPSFAVQV XXSXQXXXXX XXXXXAIE |
| 7 | PYYGKXLXXX YLQPSFAVQV XXSXQXXXXX XXXXXXAIE |
| 8 | PYYGKXLXXX YLQPSFAVQV XXSXQXXXXX XXXXXXXAIE |
| 9 | PYYGKXLXXX YLQPSFAVQV XXSXQXXXXX XXXXXXXXAI E |
| 10 | PYYGKXLXXX YLQPSFAVQV XXSXQXXXXX XXXXXXXXXA IE |

The ability to derive a unique consensus sequence which encompasses all independent isolated CD44-interacting peptides further validates the results of the two-hybrid screening method.

It has to be emphasized that the peptides of the invention show for the first time that not only large antibodies can be generated against CD44 epitopes but also rather small peptides with a length between 32 and 66 amino acids which bind specifically and selectively to a clinically relevant CD44 domain.

In contrast to the (monoclonal) CD44v5 antibodies of the prior art, these peptides exhibit several advantages.

Since they represent short peptides without the necessity to form complexes or intramolecular disulfide bridges they are much easier to produce and to purify.

As peptides with a length of between 32 and 66 amino acids it is even possible to synthesize them by solid phase synthesis (according Merrifield). This ex *vivo*-protein synthesis is especially of advantage given the strict regulatory requirements associated with recombinant protein expression.

Furthermore, as small peptides they can be advantageously conjugated or fused to a different protein, specific compounds or even nanoparticles in order to be used for therapy or diagnosis.

As small peptides they are able to cross the blood brain barrier and cellular membranes to deliver compounds to compartments which could not be addressed by antibodies.

Due to their small size the proteins of the inventions the risk to elicit an immune response is strongly reduced. It has to be remarked that the risk of immunogenicity which is inherent for antibodies markedly impairs their clinical use.

It has to be emphasized that the CD44ex9-binding protein of the invention by interfering only with the variant domain v5 leaves the normal CD44 function untouched and therefore allows a specific intervention.

In sum the CD44ex9-binding proteins of the invention open the door to novel strategies for therapy and diagnosis and cancer with a reduced risk of side effects.

### DETAILED DESCRIPTION OF THE INVENTION

In one embodiment of the invention the invention provides a protein binding to a polypeptide encoded by exon 9 of human CD44 (CD44ex9), said protein comprises or consists of an amino acid sequence with at least 95% identity and specifically at least 97.5% identity to the amino acid sequence given in SEQ ID No. 1 to 5.

In a further aspect the application describes a protein binding to a polypeptide encoded by exon 9 of human CD44 (CD44ex9), said protein comprises or consists of an amino acid sequence with at least 90% identity, preferably at least 95%, more preferably at least 97.5% and even more preferably at least 100% identity to the CD44-binding motif defined by the sequence: "PYYGKXLX₃YLQPSFAVQVX₂SXQX₁₀₋₁₄AIE" as depicted in SEQ ID Nos. 6 to 10, wherein X denotes an arbitrary amino acid.

The proteins as claimed herein have a length of 100 amino acids or less and even more preferably a length of 90, 85, 80, 75, 70 or 66 amino acids or less.

In a further embodiment of the invention the CD44ex9-binding protein has a high affinity to the polypeptide encoded by exon9 with a kᵢ value of less than 10 µM, preferably of less than 1 µM, more preferably of less than 100nM and most preferably of less than 10 nM.

In a further embodiment of the invention the CD44ex9-binding protein binds selectively to the polypeptide encoded by exon9, which is given when it binds to other arbitrary proteins with a kᵢ value which is preferably at least 10 times less and more preferably 100 times less of the ki value for CD44ex9-binding.

In one embodiment the CD44ex9-binding protein of the invention is capable to bind to every CD44 isoform that contains the domain encoded by exon 9, which is also designated as "variant 5" (v5).

In a preferred embodiment the CD44ex9-binding protein of the invention binds to a CD44 isoform selected from the list consisting of CD44 v5, CD44 v5 -v6, CD44 v3 - v6, CD44 v3 - v6, CD44 v2 - v10, CD44 v3 - v10, CD44 V4 - v7 and CD44 v4 - v10.

Notably, the CD44ex9-binding protein of the invention binds also to proteins that comprise beside the domain v5 also other variant domains such as v1, v2, v3, v4, v6, v7, v8, v9 and/or v10.

The conjugated nanoparticle according to invention can in general be linked to any type of drug which impairs, inhibits or even kills cancer cells. Hence, a cytotoxic agent is particularly preferred. Non-limiting examples for cytotoxic agents include emozolomide, carmustine, lomustine, procarbazine, streptozocin, irinotecan or any combination of two or more of these agents.

Further examples for suitable cancer inhibitors are (-)-Ci-Cdp1, (-)-Ci-Cdp2, (-)-epigallocatechin gallate, (+)-Cbi-Cdpi2, (+)-Ci-Cdp2, 10-Deacetylbaccatin III, 4-demethoxy daunorubicin, 5-azacytidine/5-aza-2'-deoxycytidine, 5-fluorouracil, 5-iminodoxorubicin hydrochloride, 6-mercaptopurine, aclarubicin, acodazole, actinomycin D, adenine phosphate, adenosine, aderbasib, adozelesin; U-73, 975, afeletecan, alemtuzumab, alitreninoin, alosetron HCl, alphitolic acid, altretamine, alvespimycin, ambazone, ametantrone, amifostine, aminoglutethimide, amsacrine HCl, amsilarotene, amygdalin, anagrelide, anastrozole, anaxirone, ancitabine, annomontacin, annomuricin A, (C19/C20-Erythro), annomuricin B, (C 1 0/C I I, C19/C20-Erythro), annomuricin C, (All Threo) annomuricin E, annonacin, annonacin-IO-One, annonacin-A-One, annonidin B, annonin VI, annosquamosin A, annosquamosin B, antramycin, apaziquone, argimesna, aristoforin, arsenic trioxide, artemisinin, ascomycin, asparaginase, atosiban, atrimustine, axitinib, azasetron HCl, azatepa, azathioprine, azotomycin, bafetinib, balamapimod, banoxantrone, batabulin, batimastat, Bbr-34384, becatecarin, belotecan, benaxibine, bendamustine, benzodepa, berubicin, betulin, betulinic acid, betulinic aldehyde, bevacizumab, bexarotene, bicalutamide, bietaserpine, biricodar, bisantrene, bistramid A; bistratene A, bizelesin, bleomycin, bleomycin A2 [Sulfate], bleomycin A5, bleomycin Sulfate, bortezomib, bosentan, bosutinib, brequinar sodium, brequinar, bropirimine, brostallicin, budotitane, bullatacin, buserelin, busulfan, cabazitaxel, calcium folinate, calcium levofolinate, calusterone, camptothecin, canertinib, canfosfamide, cantharidin, capecitabine, caracemide, carbetimer, carboplatin, carboprost (carboprost tromethamine), carboquone, carfilzomib, carglumic acid, carmofur, carzelesin, cedefingol, cemadotin, cetuximab, cevipabulin, chlorambucil, chlormethine (mechlorethamine), chlorotamoxifen, chlorotrianisene, cioteronel, cisplatin, cladribine, clanfenur, clofarabine, clofazimine, clomifene citrate, cordycepin, corosolic acid, crisnatol, curcumin, cyclocytidine, cyclophosphamide, cytarabine, cytidine, D-aminolevulinic acid, dacarbazine, damsin, daniquidone, danusertib, daporinad, darinaparsin, dasatinib, daunoblastin, daunorubicin/ daunomycin, decitabine, deferasirox, deforolimus, demecolcine, denibulin, detorubicin, dexniguldipine, dexormaplatin, dezaguanine, dianhydrodulcitolum, dibrospidium chloride, dienogest, diflomotecan, dinalin, disermolide, docetaxel, dofequidar, dolasetron mesylate, dovitinib, doxifluridine, doxorubicin, dromostanolone, duazomycin, duocarmycin, dynemicin, ecomustine, edatrexate, edotecarin, edotreotide, eflornithine, elacridar, eacytarabine, elesclomol, elinafide, elomotecan, elsamitrucin, emitefur, enloplatin, enocitabine, enpromate, entecavir, entinostat, entricitabine, enzastaurin, epirubicin, eptaloprost, eribulin, erlotinib, Esorubicin, estramustine, etalocib, etanidazole, etoglucid, etoposide, exatecan, exemestane, exisulind, fadrozole, fazarabine, fiacitabine, floxuridine, fludarabine, fluoxymesterone, fluorocitabine, flutamide, formestane, forodesine, fosfluridine tidoxil, fosquidone, fostriecin, fotemustine, fotretamine, fulvestrant, fumagillin, galarubicin, galocitabine, gefitinib, gemcitabine, gemtuzumab ozogamicin, geroquinol, gigantetronenin, gigantetroneninone, gimatecan, gimeracil, gloxazone, glufosfamide, goniothalamicin, goniothalamicinone, goserelin, granisetron HCl, gusperimus, hexarelin, homoharrlngonine, hydrocamptothecine, hydroxy carbamide, hydroxyurea, hypericin, ibandronate sodium, ibandronic acid, idarubicin HCl, idronoxil, ifosfamide, ilmofosine, imatinib, imatinib mesylate, imexon, improsulfan, incadronate, indibulin, indisulam, inolitazone, inproquone, intiquinatine, intoplicine, iobenguane, irofulven, irsogladine, ispinesib, ixabepilone, ketotrexate, L-alanosine, laniquidar, lapatinib ditosylate, laromustine, larotaxel, ledoxantrone, lenalidomide, lentinan, lestaurtinib, letrozole, leuprolide acetate, leuprorelin, lexacalcitol, liarozole, lobaplatin, lonafarnib, lonidamine, losoxantrone, Ly-83583, lysipressin, mafosfamide, mannomustine, mannosulfan, marimastat, marinomycin A, masitinib, maslinic acid, masoprocol, mechlorethamine, medorubicin, megestrol, mepitiostane, mercaptopurine, mesna, methotrexate, methyl aminolevulinate, metomidate, metoprine, meturedepa, miboplatin, midostaurin, mifamurtide, milataxel, miproxifene, miriplatin, misonidazole, mitindomide, mitoflaxone, mitoguazone, mitomycin, mitonafide, mitoquidone, mitotane, mitoxantrone, mitozolomide, mivobulin, mizoribine, mofarotene, mopidamol, motesanib, motexafin, mubritinib, muricapentocin, muricatacin, mustine HCl, mycophenolate mofetil, mycophenolic acid, nedaplatin, nelzarabine, nemorubicin, neocuproine, neptamustine, neratinib, nigericin, nilotinib, nilutamide, nimustine, ninopterin, nitracrine, nogalamycin, nolatrexed, norcantharidine, nor-dihydroguaiaretic acid, nortopixantrone, novembichin, obatoclax, octreotide, olaparib, oleanolic aldehyde, omacetaxine mepesuccinate, ombrabulin, omtripolide, ondansetron HCl, ortataxel, oteracil, oteracil potassium, oxaliplatin, oxisuran, oxophenarsine, paclitaxel ceribate, palifosfamide, palonosetron, pamidronate disodium, pamidronic acid, panitumumab, panobinostat, patubilone, pazelliptine, pazopanib, pegaspargase, peldesine, pelitinib, pelitrexol, pemetrexed disodium, pentostatin, peplomycin, peretinoin, perfosfamide, perifosine, pibrozelesin hydrobromide, picoplatin, pinafide, piposulfan, pirarubicin, pirfenidone, piritrexim, piroxantrone, pixantrone, plevitrexed, plicamycin, plitidepsin, plomestane, podophyllotoxin, pomalidomide, porfimer sodium, pralatrexate, prinomastat, procarbazine HC1, propamidine, prospidium chloride, pumitepa, puromycin, pyrazofurin, ouarfloxin, raltegravir, raltitrexed, ramosetron HC1, ranimustine, retaspimycin, retelliptine, riboprine, ritrosulfan, rituximab, roflumilast, romidepsin, ropidoxuridine, roquinimex, rosabulin, rubitecan, sabarubicin, safingol, salirasib, sapacitabine, saracatinib, sardomozide, satraplatin, sebriplatin, seliciclib, semaxanib; SU-5416, semustine, sermorelin, simotaxel, simtrazene, sitagliptin, sizofuran, soblitodin, sobuzoxane, sodium phenylbutyrate, sorafenib, sparfosic acid, sparsomycin, spiroplatin, squalamine, squamocin, streptonigrin, streptovarycin, sufosfamide, sulofenur, sunitinib, swainsonine, tacedinaline, tafluposide, talabostat, talisomycin, tallimustine, talotrexin, taltobulin, tamoxifen citrate, tandutinib, tanespimycin, tariquidar, tasidotin, tasisulam, tauromustine, tegafur, tegafur-uracil, telantinib, teloxantrone, temozolomide, teniposide, tenuazonic acid, terameprocol, teriparatide, tesetaxel, testolactone, tezacitabine, thiamiprine, thioguanine, thiotepa, thymopoietin, tiazofurine, tilomisole, tilorone, timcodar, timonacic, tioguanine, tirapazamine, tocladesine, tomudex, topotecan hydrochloride, toremifene citrate, tosedostat, tositumomab, toxipantrone, trastuzumab, trenimon, tretinoin, triciribine, trilostane, trimetrexate, triplatin tetranitrate, triptolide, triptorelin, trofosfamide, tropisetron HC1, tubulozole, tylophorin, U-67786, U-68415, U-71184, U-76074, U-78057, ubenimex, uramustine, uredepa, urethane, uridine, ursolic acid, ursolic aldehyde, vadimezan, valrubicin, valspodar, vandetanib, vapreotide, vatalanib; PTK-787, verteporfin, vildagliptin, vinblastine sulfate, vincristine, vindesine, vinepidine, vinflunine, vinformide, vinfosiltine, vinleucinol, vinleurosine, vinorelbine [Base], vinorelbine tartrate, vintriptol, vinzolidine, voriconazole, vorinostat, vorozole, wilforlide A, xanthomycin A, zalcitabine, zeniplatin, zilascorb, zinostatin, zoledronic acid, zorubicin, zosuquidar, or the like, or a combination comprising at least one of the foregoing cancer inhibitors.

Furthermore, also an anti-angiogenic drug could be used for the conjugated nanoparticle of the invention. Non-limiting examples are bevacizumab, aflibercept, cediranib, sorafenib, sunitinib, vandetanib, pazopanib, vatalanib, imatinib mesylate, cilengitide, angiostatin, endostatin, platelet factor-4. Particularly, said anti-angiogenic drug could be used in any combination of two or more of the listed examples.

The radiation sensitizing agents represents a further drug class for the conjugated nanoparticle comprising the CD44ex9-binding protein according the invention. Non-limiting examples are carboplatin, cilengitide, CG841251, staurosporine derivatives such as MK-1775, 4-phenylbutyrate, a gadolinium containing compound such as motexafin-gadolinium, a taxane derivative such as paclitaxel or docetaxel or a combination thereof.

In another aspect the application discloses an isolated nucleic acid molecule encoding the CD44ex9-binding protein or the CD44v5 peptide according to the invention.

In a further aspect the application discloses an expression vector containing the nucleotide sequence of the CD44ex9-binding protein, the CD44v5 peptide or a fusion protein thereof.

In a still further aspect the application discloses a host cell containing the expression vector containing the nucleotide sequence of the CD44 ex9 protein, the CD44v5 peptide or a fusion protein thereof.

In a further aspect the application discloses a method of producing the CD44ex9-binding protein or the CD44v5 peptide, wherein said method comprises the following steps:
1.) Transforming a host cell with an expression construct comprising a nucleic acid molecule encoding the protein of the invention or a respective fusion protein; and
2.) Culturing the host cell under conditions suitable for producing the CD44ex9-binding protein or the CD44v5 peptide.

In one embodiment of the invention the conjugated nanoparticle of the invention can be used for diagnosis or treatment of a disease selected from the group consisting of autoimmune diseases such as insulin-dependent diabetes, multiple sclerosis, Sjögren's syndrome or systemic lupus erythematosus (SLE); skin diseases such as psoriasis or atopic dermatitis; chronic inflammatory diseases such as rheumatoid arthritis or inflammatory bowel disease; tissue injury; allergic diseases and cancer disease.

The use of the conjugated nanoparticle of the invention for the diagnosis or treatment of inflammation is based on the fact that CD44 plays a role in directing inflammatory cells to the site of infection or tissue destruction. Hereby the CD44 on activated T-lymphocytes, monocytes or activated endothelial cells binds the extracellular matrix component hyaluronan, induces chemokines such as CCR2 which then stimulates the migration of inflammatory cells to the inflammatory site (Johnson & Ruffell, 2009). Blockage of CD44 by the CD44ex9-binding protein or blockade of the hyaluronan by CD44v5 peptide would prevent this process.

For example CD44 expression has been extensively studied in patients with rheumatoid arthritis whereby the level of CD44 expression on monocytic cells in the synovial fluid from patients with RA has been shown to be elevated. This increase in CD44 expression was positively correlated with the degree of synovial inflammation in RA. Furthermore, CD44 deficient mice have lower grades of arthritis and anti-CD44 treatment was shown to effectively reduce the arthritis score in animal models. Hence, the CD44-ex9-binding protein or the CD44v5 peptide of the invention can be used for diagnosis or therapy of RA.

In a further embodiment of the invention the conjugated nanoparticle can be used for producing immunosuppression, e.g. for prevention of transplant rejection by inhibiting T-cell proliferation.

In a preferred embodiment of the invention the conjugated nanoparticle can be used for diagnosis or treatment of cancer disease. This preferred use is based on the fact that CD44 variants comprising the v5 domain have been described in cancer cells.

Due to the specific binding of the CD44ex9-binding protein towards the v5 domain of the CD44 protein, said binding protein inhibits the interaction of CD44 with various extracellular matrix proteins and thus inhibits the attachment, migration and metastasis of tumor cells and may also impair the proliferation rate of tumor cells.

In a further preferred embodiment the conjugated nanoparticle of the invention can be used for the diagnosis or treatment of cancer stem cells (CSC). An emerging concept indicates that CSC ultimately determine the success of cancer treatment since the cells represent a long term surviving population which later on leads to relapses and metastasis (Deonarain et al., 2009). CD44 was shown to be expressed on CSCs and was made responsible for said long term survival and metastatic potential of the respective tumors. As an example the CSC of breast cancer are characterized by the expression of CD44⁺/CD24^{low}, which argues for CD44 as a promising anti CLC-target.

In another embodiment of the invention the conjugated nanoparticle of the invention can be used for the diagnosis or treatment of chronic lymphatic leukemia (CLL). Zhang et al (2013) could show that CLL cells show a high expression of CD44 and furthermore that anti CD44 antibodies were effective in killing CLL-cells in vitro and in vivo, whereby in a xenograft model the tumor completely disappeared from the organism.

The diagnosis or treatment of breast cancer by the conjugated nanoparticle of the invention is supported by the fact that CD44v5 represents the most abundantly expressed CD44 variant in breast cancer (56% for CD44v5 vs. 24% for CD44v6 and 15% for CD44v8). Furthermore the expression of the CD44v5 variant is associated with a shorter survival time of breast cancer: The five year survival time is 71% in CD44v5 cancer patient versus 86% in CD44v5 negative patients (Tempfer et al. 1996).

In a preferred embodiment the conjugated nanoparticle of the invention can be used for the diagnosis or treatment of colorectal cancer, as it was demonstrated that CD44v5 (and CD44v6) are associated with a poor prognosis in colorectal cancer. Hereby, patients with higher CD44v5 or CD44v6 content in tumor samples had a considerably shorter relapse-free survival (Vizoso et al. 2004).

In another preferred embodiment the conjugated nanoparticle of the invention can be used for the diagnosis or treatment of head and neck squamous cell (HNSCC) tumors, as these tumors exhibit a high expression of CD44 and the subpopulation with high CD44 expression are less sensitive to radiation and chemotherapy (La Fleur et al, 2012).

In a further embodiment the conjugated nanoparticle of the invention can be used for the diagnosis or treatment of melanomas, as a CD44-derived peptide showed in vitro and in vivo efficacy in a melanoma tumor model (Piotrowicz et al,. 2011).

In a preferred embodiment of the invention the conjugated nanoparticle is used for preparing a contrast agent for medical use.

In a further preferred embodiment the contrast agent is capable to identify cancer cells or carcinoma in situ cells.

In a specific embodiment the conjugated nanoparticle of the invention is capable to identify cancer cells which are selected from the list consisting of adenocarcinoma cells, thymic epithelial tumor cells, cervical carcinoma cells, non-Hodgkin lymphoma cells, lung cell carcinoma cells, pancreas carcinoma cells and cancer stem cells.

The CD44ex9-binding proteins of the invention may be bound to nanoparticles of any kind. Several classes of nanoparticles are known in prior art and the skilled person can select the appropriate type of nanoparticle according to the specific therapeutic or diagnostic requirements.

Examples for nanoparticle to be coupled with the CD44ex9-binding protein are quantum dots, Noble metal clusters, superparamagnetic iron oxide nanoparticles (IONPs), block-copolymer micelles, nanocells, dendrimers, nanotubes, polymersomes, XPclad® nanoparticles, and nanoparticles consisting of amorphous silica surrounded by a crystalline luminescent calcium phosphate layer (e.g. ORMOBEAD®).

The ORMOBEAD particles can be suitably modified on the surface with polyethylene imine or TRIAMO yielding amine groups or 6-amino hexanoic acid (AHA) or with adipic acid yielding carboxyl groups. These groups can be used for the coupling to the proteins of the invention. The ORMOBEAD technology is disclosed by Dembski et al. (2013) together with methods of preparing and using said nanoparticles.

In one embodiment of the invention SiO₂/Zn₂SiO₄:Mn²⁺ and SiO₂/Ca₁₀(PO₄)₆OH:Eu³⁺ core-shell nanoparticles with diameters below 100 nm are used as nanoparticles for coupling with the proteins of the invention. These particles are disclosed by Dembski et al. (2011 a, 2011 b) together with methods of preparing and using said nanoparticles.

In a further embodiment luminescent dye-labeled hybrid nanoparticles can be used. These nanoparticles consist of a SiO₂-based particle matrix with covalently attached organic fluorophores. They combine the optical properties of organic dye molecules and the inorganic particle matrix properties. As a result they show an increased resistance to photobleaching and a decreased dye leakage. Respective nanoparticles are disclosed by Probst et al. (2012) together with methods of preparing and using said nanoparticles.

In another embodiment, cadmium-free quantum dots can be used. These nanoparticles show bright emission in the visible and near infra-red region of the spectrum. Respective nanoparticles are developed by Nanoco Technologies Ltd. (Manchester, UK) and are disclosed in WO07/020416 A1, WO08/100276 A2, WO10/52455 A1, WO10/15824 A1, WO10/10329 A2 and WO13/93631 A2 together with methods of preparing and using said nanoparticles.

In one embodiment of the invention (group II-alloyed) group I-III-VI semiconductor quantum dots, group III-V quantum dots or micronized semiconductor nanocrystal complexes as developed by Evident Technologies (Troy, NY, USA) can be used. These nanoparticles are disclosed in WO07/118118 A2, WO08/94292 A1, WO06/17125 A2 or WO05/110916 A2together with methods of preparing and using said nanoparticles.

In another embodiment superparamagnetic iron oxide nanoparticles (IONPs), block-copolymer micelles, nanocells, dendrimers, nanotubes, polymersomes and XPclad® nanoparticles can be used. Respective nanoparticles are disclosed by Singh and Lillard (2009) and Xie et al. (2010) together with methods of preparing and using said nanoparticles.

In a further embodiment of the invention non-Cd-nanoparticles can be used, comprising a core area being covered by a shell area which represents an antireflective coating of the core area. Respective nanoparticles are disclosed by US 2008/0286826 A1 (Philips Intellectual Property & Standards) together with methods of preparing and using said nanoparticles.

In another embodiment of the invention, magnetic particles can be used, which are especially suited for targeted drug delivery. In a preferred embodiment said magnetic particles consist of superparamagnetic metal oxides and/or metals and are coated with the peptides of the invention and optionally with one or more additional drugs. Respective magnetic particles are disclosed by EP 1 267 843 A1 (EUCRO) together with methods of preparing and using said magnetic particles.

The modified nanoparticles may preferably be employed as in vivo contrast agents for detecting CRC cells. WO 2007/057182 A2 discloses advantageous nanoparticles, methods of preparing and using said nanoparticles. Said nanoparticles are in particular those whose hydrodynamic diameter does not exceed 15 nm and which are non-inert in biological systems.

The nanoparticles of the invention can be further conjugated to at least one tumor antigen-binding substance and/or cytotoxic agent.

In a preferred embodiment of the invention the nanoparticle is further conjugated to a tumor antigen-binding substance which is selected from the list consisting of an antibody against CEA, an antibody against CA-19-9, and an adhesin or any combination thereof.

In a preferred embodiment the adhesin conjugated to nanoparticle is modified in its amino acid sequence, and more preferably modified according WO 2009/106102 A1.

The preferred nanoparticles of the invention comprise at least three structures, namely an inorganic core which is coated by a layer comprising an imidazole component containing layer (which in the following is also referred to as a "passivation layer") which then in turn carries specific ligands, wherein said specific ligands may also be part of the layer. Said ligands result in the nanoparticle binding specifically to the target of the biological system.

In preferred nanoparticles, the inorganic core including the passivation layer surrounding it has a hydrodynamic diameter of no more than 15, if possible, preferably no more than 10 nm. Particular preference is given to hydrodynamic diameters of no more than 8 nm or no more than 5 nm. This applies especially to spherical nanoparticles. Nanoparticles of this size can be illuminated via the kidneys and therefore do not accumulate, or at most accumulate in tolerable quantities, in the body. This makes *in vivo* application possible. This applies in particular to nanoparticles having a hydrodynamic diameter of no more than 5 nm.

In an alternative embodiment, the nanoparticles may also be rod-like. In this embodiment, it is advantageous if the diameter of the rod does not exceed the abovementioned limit of 15 nm. Here too, preference is given to diameters in the range of 5, 8 or 10 nm to facilitate elimination from the body. Thus, for example, the nanoparticles employable according to the invention may have length/breadth dimensions of 8 x 15 nm.

The nanoparticles employable according to the invention preferably have maximum emission at a wavelength between 600 and 700 nm, for example between 620 and 660, particularly preferably at about 625 nm or 655 nm. Said emission is readily visible to the human eye, and such nanoparticles can therefore be used directly as contrast agents for medical interventions. Consequently, auxiliary optical instruments may in some circumstances be dispensed with.

In an alternative embodiment, nanoparticles exceeding the abovementioned hydrodynamic diameters may be employed according to the invention, as long as the particles are guaranteed to be non-inert *in vivo.* The latter is the precondition for said particles to be biodegradable and, as a result, the metals (e.g. Cd) which are bound therein as particulates initially, to be converted into the ionic form. The degradation products can be illuminated via the kidneys.

Inorganic nanoparticles having a passivation layer containing an imidazole component are indeed non-inert *in vivo,* as has been demonstrated previously, but they are degraded under these conditions. Said nanoparticles therefore satisfy the criterion of biodegradability and of renal passage of the degradation products, which is particularly relevant for *in vivo* application. This was a surprise finding because the passivation layer serves especially also to increase the chemical and/or physical stability of the nanoparticles (in this context, see also the additional comments below). Thus, the relationship between on the one hand the stability of the nanoparticles which is required for good diagnostics, and on the other hand biodegradability which is required for renal passage of "large" particles is suitable for use as *in vivo* contrast agent.

The main task of the passivation layer is to increase fluorescence intensity and chemical and physical stability of the inorganic core. The inorganic cores coated by the passivation layer are characterized by a quantum yield of at least 10%, advantageously at least 30, 50 or even 70%. Quantum yield here means the ratio of the amount of the light emitted by a sample to the amount of light absorbed by the sample. Advantageously, the passivation layer has a thickness of no more than 1 nm. In this case, the diameter of the passivated core increased by no more than 2 nm.

Advantageously, the nanoparticles are in each case also provided with modifiers, in particular for improving compatibility with the biological environment. Preferably, the increase in the hydrodynamic radius due to the use of modifiers does not exceed 2 nm. In particular cases, the thickness of the passivation layer and the modifiers also depends on the relationships of the two structures among each other and in relation to the inorganic core.

The preferably used nanoparticles of the invention, if restricted in size as mentioned above, are particularly suitable for the use as diagnostic agent in a living patient. Thus, the size reduction increases the rate of diffusion and depth of penetration into the tissue. This allows the nanoparticles to spread evenly and rapidly in the biological environment and also penetration as far as possible of a tissue (for example a tumor) after local administration. The nanoparticles of the invention likewise allow systemic administration which may also be carried out by way of injection. However, local administration, for example topical application or intra- or peritumoral administration for the treatment of tumors is also possible.

Particularly advantageous embodiments of the invention comprising the nanoparticles coupled to the proteins of the invention have a hydrodynamic diameter of no more than 8, particularly preferably of no more than 4 nm. Nanoparticles of this order of magnitude may already be illuminated via the kidneys and therefore do not accumulate, or accumulate to a distinctly lesser extent, in the body. As a result, the nanoparticles of the invention reduce considerably the problem of long-term toxicity probably associated with the known quantum dots.

The nanoparticles advantageously emit a fluorescent spectrum between 600 and 700 nm, particularly preferably with maximum emission between 600 and 660 nm, particularly preferably between 620 and 660 nm. Said emission spectrum has the advantage of very high tissue transmission owing to only low absorption by hemoglobin and other light-absorbing substances in a living system (including water). Light of these wavelengths can still be sensed by the human eye and therefore enables the physician in charge of the treatment to identify the labeled tissue without any further complicated technical detection aids (e.g. CCD cameras). This is particularly advantageous when using the nanoparticles of the invention as contrast agents during surgical intervention for identifying CD44, CEA- and/or CA19-9-expressing cells, in particular for discriminating carcinogenic and healthy tissues.

In one embodiment, the preferably employable nanoparticles are known nanoparticles having a core of, for example, CdSe, CdS or CdTe, as described, for example, in US 2004/0247861 A1 with reference to scientific publications. This printed publication also makes reference to documents regarding the preparation of the core materials, for example to US 6,179,912 B1. Reference is made to the entire contents of these documents regarding the disclosure of the properties of these known nanoparticles and the preparation thereof. A method of preparing nanoparticles is furthermore also disclosed in US 7,147,712 B2-

Particularly advantageously, the inorganic core of the nanoparticles essentially consists of semiconductors. These cores emit light of various colors, depending on their individual size and/or composition, but all of them absorb over a broad band within the same range of the light spectrum (UV to VIS range). Due to the high Stokes shift, excitation and emission spectra are far apart, enabling simple and simultaneous excitation of various nanoparticles. They have narrow and symmetric emission spectra which overlap only slightly or not at all. Other beneficial properties which are of great importance particularly for improved depth of filtration and *in vivo* labeling are the high quantum yield of up to 80% and high photostability.

Preferred nanoparticles have been disclosed, for example, in WO 2005/001889 A2. Accordingly, they comprise an inorganic core made of an alloy of at least two semiconductors which either are distributed homogeneously or for which there is in each case a concentration gradient within the alloy. In respect of the disclosure of the nature and preparation of said nanoparticles, reference is made to WO 2005/001889 A2 cited above. The cores may deviate in their size by in each case 5%.

Accordingly, the inorganic core of the nanoparticles may comprise an alloy of at least two semiconductors, wherein the core has a homogeneous composition and is characterized by a "band-gap energy" which is nonlinear to the molar ratio of the two semiconductors.

Alternatively, the core may be non-homogeneous, with the concentration of the first semiconductor gradually increasing, starting from the center of the core to the surface of the core, and the concentration of the second semiconductor gradually decreasing from the center of the core to its surface.

For both cores, at least one of the semiconductors is a group II-group VI semiconductor or a group III-group V semiconductor (the definition of groups corresponds to the groups of the Periodic Table of the Elements). For example, the alloy may be selected from the group of the following alloys: CdSeTe, CdSSe, CdSTe, ZnSeTe, ZnCdTe, CdHgS, CdHgTe, InGaAs, InGaP, GaAIAs, InGaN. These cores may moreover carry a coating of inorganic material such as, for example, semiconductors (e.g. ZnS). This additional layer is known to the skilled worker as "capping" or "shell".

Group II-group VI and group III-group V semiconductors are generally known and include, for example, CdS₁₋ₓSeₓ, CdS₁₋ₓTeₓ, CdSe₁₋ₓTeₓ, ZnSe₁₋ₓTeₓ, Zn₁₋ₓCdₓTe, Cd₁₋ₓHgₓS, Cd₁₋ₓHgₓTe, In₁₋ₓGaₓAs, Ga₁₋ₓAlₓAs and In₁₋ₓGaₓP. Preference is given to using the semiconductors CdSe₁₋ₓTeₓ, CdS₁₋ₓTeₓ, ZnSe₁₋ₓTeₓ, Zn₁₋ₓCdₓTe, Cd₁₋ₓHgₓS, Cd₁₋ₓHgₓTe, In₁₋ₓGaₓAs, In₁₋ₓGaₓP, where x is a fraction from 0 to 1.

The molar ratio of the semiconductors may be any molar ratio. However, if the alloy comprises CdSSe, preference is given to an alloy having the molecular formula CdS₁₋ₓSeₓ. If the alloy comprises CdSTe, preference is given to an alloy having the molecular formula CdS₁₋ₓTeₓ. If the alloy comprises ZnSeTe, preference is given to an alloy having the molecular formula ZnSe₁₋ₓTeₓ. If the alloy comprises ZnCdTe, preference is given to an alloy having the molecular formula of CdTe alone. In each of these cases, x is a fraction between 0 and 1.

These preferred inorganic cores of the nanoparticles may be prepared using the following steps: (i) preparation of a first solution under conditions which enable nanocrystals to form, (ii) preparation of a second solution which comprises a precursor of the semiconductors with a molar ratio under a condition which does not enable nanocrystals to form, (iii) addition of the second solution to the first solution which enables nanoparticles to form, and (iv) alteration of the conditions, which stops growth and formation of the nanocrystals. The method of preparing the cores is illustrated in WO 2005/001889 A2.

In an alternative embodiment, the inorganic core may essentially consist of a noble metal cluster which preferably comprises 2 and 27 noble metal atoms. In a preferred embodiment, the noble metal was selected from a group consisting of gold, silver, copper, platinum, palladium, osmium, iridium, ruthenium and rhodium. The cluster may have varying charges.

These cores have the advantage that they can be detected readily as individual "nanodots", using a weak mercury lamp excitation, owing to their strong absorbance and emission. The nanoparticles of the invention containing these cores can advantageously be used as fluorescent individual molecule label and mass label.

The term "noble metal" to a group of elements selected from a group consisting of gold, silver and copper, and the platinum group metals (PGM), platinum, palladium, osmium, iridium, ruthenium and rhodium. In preferred embodiments of the present invention, the noble metals are selected from the group consisting of gold, silver and copper. In a particularly preferred embodiment, the noble metal is silver or gold.

The term "cluster" relates to a compound of 2-27 atoms of a metal. Clusters are known inter alia from the fields of chemical catalysis, ceramics, semiconductor technology and material sciences. A person skilled in the art is therefore familiar with their preparation. WO 2004/003558 A1 describes inter alia the preparation of noble metal clusters and in addition contains extensive further references on this subject. More specifically, it discloses the preparation of noble metal nanoclusters associated with organic molecules. The term association here means any form of binding, independently of the chemical or physical nature of the binding (thus, for example, covalent, non-covalent, electrostatic or van der Waals binding). Reference is made to WO 2004/003558 A1 in respect of preparation of the nanoclusters as core of the nanoparticles of the invention.

The nanoparticles preferably employable according to the invention have a passivation layer which increases fluorescence intensity and improves the chemical and physical stability of the inorganic core. As a result, the nanoparticles emit light preferably with a quantum yield of more than 10%, preferably of more than 50%.

Said nanoparticles preferably have a storage stability of at least 12 months in an aqueous environment at 4°C and are, if possible, stable across a pH range from pH 5 to pH 10, preferably from pH 7 to pH 10, i.e. they exhibit deviations of less than 50% in respect of their specific spectral characteristics such as quantum yield, position of maximum emission, half-width of the emission spectrum. Preferred particles exhibit deviations of less than 10% in respect of these specific spectral characteristics.

The nanoparticles employable according to another embodiment of the invention exhibit essentially a constancy/stability of the properties of the core (including the passivation layer surrounding it) also under biological (i.e. physiological) conditions or *in vivo* over a period of at least three days. Preferred particles exhibit a constancy/stability of this kind for a period of from 7 to 14 days, wherein by way of stability retaining at least 50% of the one constancy of the properties. This information refers especially to the stability of the nanoparticles in the actual target organ. It is noteworthy that the stability of the nanoparticles in organs which have primarily catabolic function may be distinctly less stable (for example in the liver). This may even be expressly desirable.

Although the nanoparticles are stable in the above sense, they are nevertheless fundamentally degradable *in vivo* and consequently are non-inert. In this sense, "non-inert" means that at least 50% of the nanoparticles have already been degraded after 12 weeks or more post-administration. Preference is given to at least 50% degradation being detectable already after 8, 6 or 4 weeks. Detection of the particles remaining in the body includes detection in body organs and in the plasma for this purpose. Accordingly, "inert" means that more than 50%, even up to nearly 100%, of the particles are still detectable in the body of the patient after 4 weeks post-administration.

Degradability of the nanoparticles can be detected by assays which are known to the skilled worker, namely, for example, by inductively coupled plasma mass spectrometry (ICP-MS), which assays may also be supplemented by fluorescence spectrometry measurements, if the samples are suitable.

The passivation layer contains at least one imidazole component. Such a compound is capable of coordinating metal atoms or metal ions, for example zinc ions, mercury ions or cadmium ions. In a preferred embodiment, the imidazole group is in a terminal position based on the structure of the molecule. The passivation layer may furthermore have a crosslinker, or the cyclic or linear imidazole component may also act as a crosslinker. The crosslinker may be alkaline.

The coordination compounds containing metal atoms or metal ions may functionally bind to fluorescent inorganic cores by means of chelation, coordination or electron donor properties of Lewis bases and have correspondingly conjugated moieties/groups. Said molecules may moreover contain moieties which impart solubility or wettability to the cores coated with them in aqueous solutions.

The imidazole component is suitably crosslinked by a phosphine compound, being preferably an alkylphosphine compound.

The term "imidazole component" means for the purposes of the present description a heterocyclic or heteroaromatic molecule which contains at least one imidazole group (including imidazole derivatives) and which is available for binding of the inorganic core or the passivation layer having a metal such as cadmium, zinc, gallium, or a metal cation or a substrate containing such a cation. In this connection, preferably at least one imidazole group should be at a terminal position based on the structure of the molecule. The imidazole component in its functional form binds via the ring which contains delocalized molecular orbitals to the fluorescent nanocrystal. Usually, the nitrogen atoms of the imidazole ring serve as coordination ligands to functionally bind a metal ion such as cadmium or zinc.

In one embodiment, the imidazole component comprises reactive functional groups such as one or two amino acid(s), for example histidine, carnosine, anserine, baleine, homocarnosine, histidylphenylalanine, cyclo-histidylphenylalanine, 5-amino-4-imidazole-carboxamide, histidylleucine, 2-mercaptoimidazole, boc-histidine, hydrazide, histinol, 1-methylhistidine, 3-methylhistidine, imidazolysine, imidazole-containing ornithine (e.g. 5-methylimidazole), imidazole-containing alanine (e.g. (beta)-(2-imidazolyl)-L-(alpha) alanine), carzinine, histamine. These histidine-based molecules or imidazole-containing amino acids may be synthesized by generally known methods.

The term "phosphine" means for the purpose of the invention a molecule which has at least one phosphine group (including their derivatives) for binding or chelating a nonmetal such as Se, S or other nonmetals or substrates containing such atoms, and which provides at least one functional group (for example hydroxyl-, amino-, thiol-, carboxyl-, carboxamide- etc.) for reaction with neighboring molecules.

In a preferred embodiment of the invention the imidazole component is a peptide comprising at least one histidyl residue, and preferably a dipeptide containing one or two His-residues.

In a further preferred embodiment the imidazole component is a mixture of different histidyl-containing dipeptides.

Preferably, at least one phosphine group should be located at a terminal position based on the structure of the molecule. The phosphine moieties serve as coordination ligands to bind in its functional form with a fluorescent core or a compound from the shielding layer a nonmetal or ion such as Se or S.

In a preferred embodiment, the phosphine-containing compound includes one, two or more phosphine groups coupled to one another (e.g. in polymeric form) which may include hydroxymethylphosphine compounds or the like but without being limited thereto. Phosphine-containing compounds may be synthesized by generally known methods. Furthermore, alkylphosphine-containing compounds are known to possibly also have one or more additional functional groups (e.g. hydroxyl-, amino-, thiol-, carboxyl-, carboxamide-, etc.). Examples of derivatives are hydroxymethylphosphine derivatives, amides or esters, as long as said derivatization is compatible with the functions described herein of phosphine as coating.

Particular preference is given to tris(hydroxymethyl)phosphine and β-[tris(hydroxymethyl)phosphino]propanoic acid for coating the fluorescent inorganic cores of the nanoparticles of the invention. Crosslinked phosphine-containing compounds are well known to additionally be able to functionally bind to metal atoms and/or ions such as Zn or Cd. Isocyanates or alkylcyanoacrylates functionalized in this respect may furthermore be useful as crosslinkers for ligands and the formation of adducts with fluorescent cores. Said crosslinkers may also be basic.

The passivating effect of the passivation layer present according to the invention is based on the shielding of surface cadmium or zinc atoms or the like by complex formation with the imidazole component, and on the shielding of the counteratoms (Se or S or the like) via complex formation with the phosphine-containing compounds.

The passivation layer of the nanoparticles of the invention has been disclosed in US 2004/0247861 A1. This laid-open application describes the preparation of inorganic cores coated with the passivation layer, for example of quantum dots. Reference is therefore made to US 2004/0247861 A1 teaching the preparation of the passivation layer employed according to the invention and of the inorganic cores coated therewith.

The molecules of the passivation layer may furthermore have or carry chemical groups in order to bind and crosslink target molecules and cells (specific ligands). In the presence of appropriately suitable reagents such as ZnSO₄ and Na₂S, said molecules or compounds may form a passivation layer with the molecules on the fluorescent core ("capping" or "shell"). These reagents may also functionally bind to atoms or ions on the surface of the fluorescent nanocrystal and, as a result, this additional passivation layer may also be formed directly on the surface of the core.

In an advantageous embodiment, the nanoparticles of the invention may additionally have modifiers which may consist of organic and/or inorganic moieties. They are used for improving compatibility, efficacy and/or solubility of the nanoparticles in a liquid or a suspension medium, in particular in the physiological environment. This surface modification is especially advantageous for achieving very low unspecific adsorption and increased compatibility in biological systems, in particular in the human body.

One possibility is to modify the surface with polyethylene glycol (PEG) which has already been approved for particular medical applications, in particular in low molecular weight forms for the nanoparticle to maintain a small overall size. Thereby both biocompatibility and blood circulation time of the nanoparticles and also the efficiency of uptake into cells may be increased. Combining a low molecular weight PEG layer with other substances such as vitamins, for example folic acid, may achieve a lower uptake of said nanoparticles into macrophages because protein adsorption to the nanoparticles, which is reduced thereby, makes recognition of said nanoparticles by the immune system more difficult.

Another possible advantageous surface modification by using modifiers is the coating with monosaccharides, di- or trisaccharides at up to low molecular weight polysaccharides composed of one type of monosaccharide or different monosaccharides. One possible type of development is a modification with polyglucose, for example, in which dextran can be used which has been proved medically as blood substitute. It exhibits good biocompatibility/ tolerance. Another embodiment is the use of stereoisomeric forms (D-/L-) of saccharides in order to counteract possible degradation.

Another embodiment is the use of biologically compatible hydrophilic vitamins as modifiers, for example thiamine, riboflavin, niacin, pyridoxine, cobalamin, panthothenic acid, ascorbic acid and folic acid. Thus, for example, folic acid can lead to a preferred binding of nanoparticles to cancer cells. This vitamin exhibits only low immunogenicity and therefore high biocompatibility. Internalization of the nanoparticles is facilitated by binding to the membrane-bound folic acid receptor.

Surface modifications are also possible with lipophilic vitamins such as retinol, cholecalciferol, tocopherol and phylloquinone. Thus, for example, vitamin E can increase the cellular uptake of nanoparticles.

Fatty acids such as, for example, 1-octadecene or 18-methyleicosanoic acid and their derivatives, may increase solubility and stability of the colloids and have a terminal functional carboxyl group which may be utilized for subsequent binding of specific ligands. It is therefore useful to include fatty acids as modifiers.

Another embodiment of surface modification is a coating with polyalcohols such as, for example, diethylene glycol (DEG), which are particularly good at reducing unspecific protein adsorption. The same applies to polytetrafluoroethylene (PTFE, Teflon™), in particular in its low molecular weight forms, which can achieve reduced protein adsorption. Polytetrafluoroethylene is frequently used in cardiosurgical applications.

Surface modifications can likewise be carried out using one or more naturally occurring amino acids which include both proteinogenic and non-proteinogenic amino acids, and synthetic amino acids. Both stereoisomers (D- and L-forms) may be used here. Di-, tri-, tetra-up to small polypeptides of the abovementioned amino acids hardly stimulate the immune system and are therefore likewise suitable for a thin compatibility layer. They may be artificial amino acid sequences as well as sequences from biological proteins. Peptide derivatives of natural proteins such as, for example, phytochelatin, may likewise be used for surface modification. Surface modification with Tat peptide and Tat peptide-containing peptides is another possibility of making nanoparticles available for the use in biomedical applications. The Tat peptide is an effective molecule, for example, for delivering gold nanoparticles through the cell membrane all the way into the nucleus.

Another embodiment of possible modifiers is the formation of a phosphorylcholine coating. Phosphorylcholine reduces a possible unspecific protein adsorption, for example on contact lenses. Owing to its non-thrombogenic properties, a phosphorylcholine modification can readily be employed in biological systems and is distinguished by high storage stability.

Since polylactate is biocompatible, this substance is used in a variety of medical applications. More specifically, low molecular weight forms of polylactate constitute another possible surface modification of the nanoparticles of the invention. Both stereoisomers (D-/L-forms) may be employed here in order to reduce possible biodegradation.

Apart from the surface modifications mentioned, proteolytically cleavable binding of unspecific proteins to the nanoparticles is also possible. This may increase biocompatibility/compatibility. At the target location, the large protein may be removed with the small nanoparticles being released in the tissue. Said removal may also take place after an appropriate dwell time. Suitable for this are preferably commonly used proteins such as, for example, transferrin, lactoferrin, ceruloplasmin, elastin and albumin in addition to other proteins which reduce unspecific adsorption. Thus, for example, surface coating composed of combinations of polypeptides with elastin may prevent undesired clot formation and therefore increase the biocompatibility of the nanoparticles.

The major serum protein albumin may reduce non-specific interactions with plasma membranes. Furthermore, the appropriately modified nanoparticle retains the ability to develop specific interactions with target cells by a specific ligand simultaneously binding to the particle surface. A coating with serum albumin may result in a substantially longer blood circulation time by preventing a rapid uptake by microphages after intravenous administration, than is the case with uncoated nanoparticles.

The combination of reduced hydrodynamic diameter which results in the higher rates of diffusion and perfusion mentioned, together with the properties and improvements described above and the high fluorescence intensity, especially in the visible red light range, renders the nanoparticles of the invention a simple diagnostic agent which can be employed in many different ways for selective and accurate discrimination of tissue forms *in vivo.* These possibilities, in combination with antigen-specific biomarkers, are used especially for identifying CD44v5, CEA- and/or CA-19-9-expressing cells, in particular for distinguishing abnormal, (pre-)carcinogenic tissue from normal tissue, assisting in the visual assessment during surgical intervention for a more precise tumor resection.

The inventive nanoparticles employable herein therefore serve as contrast agents. This applies in particular to the use in cancer diagnosis and surgery.

The nanoparticles carrying the CD44ex9-binding protein according to the invention may be employed either as *in vitro,* as *ex vivo* or as *in vivo* diagnostic agent, theranostic agent and/or therapeutic agent. For this purpose, they may be administered locally (e.g. intratumorally, intramuscularly or into surgically accessible tissues/organs) or else also systemically (e.g. intravenously). Local/topical administration may be provided for by way of a liquid, spraying solution, foam, cream or active patch. This may be preferred in particular for the treatment/diagnosis of hollow organs such as in the case of bowel cancer. Oral intake is also possible, for example as syrup or in the form of tablets or capsules. Inhalation is equally possible (e.g. spray). Anal administration by suppository is envisaged. In one variant, the nanoparticles may be implanted in depot form.

In a preferred embodiment of the invention the CD44ex9-binding protein may be used for targeted drug delivery. In the context of the present invention, targeted drug delivery is defined as a measure to concentrate the medication in the tissues of interest while reducing the relative concentration of the medication in the remaining tissues. For this purpose, the CD44ex9-binding protein conjugated to a nanoparticle is bound to a drug delivery vehicle. There are numerous types of drug delivery vehicles which are known to the skilled person and which he can select according the specific purpose, such as, e.g. polymeric micelles, liposomes, lipoprotein-based drug carriers, nano-particle drug carriers, dendrimers etc. An ideal drug delivery vehicle must be non-toxic, biocompatible, non-immunogenic, biodegradable and avoids recognition by the host's defense mechanisms.

In a preferred embodiment of the invention liposomes comprising lipid-derivatized bisphosphonic acids can be used for targeted drug delivery, especially for a delivery to bone structures. Respective lipid-derivatized bisphosphonic acids are disclosed by WO 2005/070952 A2 (MCS Micro Carrier Systems GmbH) together with methods of preparing and using said bisphosphonic acids.

In a preferred embodiment of the invention the conjugated nanoparticle of the invention may be used as *ex vivo* or *in vitro* diagnostic agent. The in vitro or ex vivo diagnostic use relates to the detection of the CD44v5 protein in a sample derived from a patient. The sample to be analysed can be of any type including tissues, organs and biopsies therefrom or body fluids such as blood, serum, urine, saliva or cerebral liquor.

For the diagnostic use, the conjugated nanoparticle is labeled for detection. Appropriate labels are known in prior art and include dyes such as luminescent or fluorescent dyes, a radioactive isotope, an enzyme, a protein tag such as a FLAG tag, a GST-tag, an MBP-TAG or a His-tag, or a nanoparticle.

For the incorporation of a label several prior art methods are known which rely on the random modification of sulfhydryl, amine, or carboxyl groups. Alternatively, genetic engineering can be used to introduce or remove additional reactive groups, such as a cysteine residue.

As an alternative method a short peptide tag can be attached to the protein that functions as a label acceptor site. Here the label is attached either through an intrinsic activity of the tag or the activity of an enzyme supplied in trans. Examples for said activity-associated labeling include site-specific biotinylation, as mediated by the biotin holoenzyme synthetase BirA which allows tagged proteins to be combined with a broad range of avidin/streptavidin reagents. Proteins fused to the LUMIO-tag (a hexapeptide containing a tetracysteine motif) can be stained directly with dyes containing two arsenic atoms.

In a preferred embodiment of the invention, the engineered version of the human DNA-repair enzyme *O*(6)-alkylguanine DNA alkyl transferase (AGT; also known as SNAP-Tag) is fused to the CD44ex-binding protein. Substrates containing *O*(6)-benzylguanine are covalently bound to the fusion proteins via a stable thioether bond in a rapid and highly specific self-labeling reaction. This label technique and its use for diagnostic purposes is disclosed by Kampmeier et al. (2009) and Xie et al. (2010) together with methods of preparing and using said labels.

According to the invention, the CD44ex9-binding protein is bound to a nanoparticle. Various types of nanoparticles are known in prior art and also disclosed in the present application.

In a further preferred embodiment, the labeled CD44ex9-binding protein, which is bound to a nanoparticle, is contained within an aqueous immersion bath. By dipping the sample into the immersion bath, the labeled CD44ex9-binding protein will specifically label the CD44v5 proteins of the sample which can be directly assessed visually by the investigator. This procedure is especially suited for surgery, whereby the withdrawn tissue can be analysed during surgery for the presence of tumor markers. As a result the surgeon has an immediate feedback regarding the precision of tumor resection.

The term "diagnostic agent" is used in the context of the present invention as a synonym for "contrast agent", i.e. it serves for the discriminating visualization of morphological or functional structures in biological systems, especially in living people, to assist a medical intervention.

The nanoparticles may be employed as diagnostic agent especially in surgical interventions. They can likewise be used in minimally invasive methods (e.g. endoscopy, laparoscopy). Combination with imaging methods such as PET, MRT, CT, etc. is worthwhile.

As already stated above, the use according to the invention in the form of local administration is particularly advantageous. The amount of Cd employed on local administration in this connection advantageously does not exceed one tenth of the total exposure which normally accumulate anyway during the course of life in the liver and kidney of a person of advanced age and usual lifestyle. The total exposure of these organs is about 18 mg. Accordingly, it is advantageous on local administration for the amount of nanoparticles to be limited so that the amount of Cd supplied at least does not substantially exceed 2 mg. In a particularly preferred embodiment, tumor visualization is possible even with an amount of contrast agent which does not exceed a total amount of 0.6 mg, particularly preferably 0.2 mg, of cadmium.

"Local administration" means for the purpose of the invention any administration, on which an increased amount or dose of the contrast agent can be expected in distinct regions of the body depending on the manner of administration. Accordingly a vascular administration of the contrast agent is also a local administration, if accompanying measures by the administering personnel, such as, for example, applying a ligature to afferent or efferent vessels, prevent the contrast agent from spreading across the blood vascular system in the body in an essentially unimpeded manner.

The particular advantage of this embodiment is that the use of the nanoparticles in medical application on a living person is thereby possible because otherwise - i.e. as systemic administration - this is precluded because of the toxicity associated therewith. This is because local administration reduces the dose of nanoparticles necessary for adequate visualization.

It has emerged that the Cd-containing contrast agent is advantageously employed according to the invention for visualizing a tumor *in vivo* in a dose corresponding to an amount of from 0.002 to 0.02 mg of Cd per cm³ of tumor tissue. Dosages of the contrast agent of from 0.002 to 0.015 mg of Cd/cm³ of tumor tissue are particularly advantageous, in particular those between 0.002 and 0.010 mg of Cd/cm³. It is possible with this advantageous dosage to visualize tumors with a volume of up to about 150 cm³ *in vivo* without thereby exceeding the normally acceptable upper limit of exposure for humans. The visualization of tumors with a volume of up to 50 cm³ is particularly favorable.

The investigations may relate to all accessible tissues/organs of the patient, especially in the skin, hollow organs (e.g. in the gastrointestinal, urogenital, respiratory tract) or else externally accessible regions of the sensory organs and also the cardiovascular system.

Use as an *in vitro* diagnostic agent is also possible, for example immunohistochemistry or FACS, and ELISA. A combination of *in vivo* and *in vitro* diagnosis (e.g. biopsy material) is particularly advantageous.

The CD44ex9-binding proteins according to the invention may remain bound to the nanoparticles or may be removable or detectable or releasable.

In a further aspect the application discloses CD44 v5-peptides as listed in the following table and designated as SEQ ID No. 11 to 38. These peptides are 12-mers with overlapping sequences covering the complete v5 domain of human CD44.

| CD44v5-Peptide No. | Amino acid sequence | SEQ ID No. |
|---|---|---|
| 1 | DVDRNGTTAYEG | 11 |
| 2 | VDRNGTTAYEGN | 12 |
| 3 | DRNGTTAYEGNW | 13 |
| 4 | RNGTTAYEGNWN | 14 |
| 5 | NGTTAYEGNWNP | 15 |
| 6 | GTTAYEGNWNPE | 16 |
| 7 | TTAYEGNWNPEA | 17 |
| 8 | TAYEGNWNPEAH | 18 |
| 9 | AYEGNWNPEAHP | 19 |
| 10 | YEGNWNPEAHPP | 20 |
| 11 | EGNWNPEAHPPL | 21 |
| 12 | GNWNPEAHPPLI | 22 |
| 13 | NWNPEAHPPLIH | 23 |
| 14 | WNPEAHPPLIHH | 24 |
| 15 | NPEAHPPLIHHE | 25 |
| 16 | PEAHPPLIHHEH | 26 |
| 17 | EAHPPLIHHEHH | 27 |
| 18 | AHPPLIHHEHHE | 28 |
| 19 | HPPLIHHEHHEE | 29 |
| 20 | PPLIHHEHHEEE | 30 |
| 21 | PLIHHEHHEEEE | 31 |
| 22 | LIHHEHHEEEET | 32 |
| 23 | IHHEHHEEEETP | 33 |
| 24 | HHEHHEEEETPH | 34 |
| 25 | HEHHEEEETPHS | 35 |
| 26 | EHHEEEETPHST | 36 |
| 27 | HHEEEETPHSTS | 37 |
| 28 | HEEEETPHSTST | 38 |

In one aspect, the application discloses a peptide as designated as SEQ ID No. 11 to 38 having an N-terminal and/or C-terminal deletion of one, two or three amino acids.

In another aspect, the application discloses also a peptide as designated as SEQ ID No. 11 to 38 having an N-terminal and/or C-terminal extension of one, two or three amino acids, whereby these additional amino acids are those of the respective CD44v5 amino acid sequence.

In another aspect, said peptide can be used for blocking the conjugated nanoparticle of the invention. Due to this antagonistic activity, these peptides could be used to verify the specificity of an *in vitro, ex vivo* or *in vivo* CD44 labelling.

Furthermore, said peptide could be used as antagonist for the therapeutic use of the conjugated nanoparticle. In this context the peptide could be used to regulate, diminish or cancel the effect of the CD44ex9-associated therapy.

In a therapeutic setting the CD44v5 peptides could directly bind to the endogenous ligands of the CD44 receptor. This could lead to neutralization of these ligands (making them unavailable for CD44 receptors) and could also lead to a rapid clearance by macrophages.

In a further aspect the application discloses that one or more of these peptides according SEQ ID No. 11 to 38 could be used as a vaccine in order to stimulate the immune system of the individual to develop adaptive immunity against the CD44 variants expressing the v5-domain. As a vaccine said peptides could be used for prevention of cancer or for an immunotherapy of cancer.

Due to successful outcome of the two hybrid screening leading to the identification of the CD44ex9-binding protein, the respective peptides have been validated as immunologically relevant epitopes. Hence, they are suitable for the use as vaccines.

The application also discloses a vaccine composition comprising an isolated protein according SEQ ID No. 11 to 38 or a peptide fragment hereof or a nucleic acid encoding said protein or said peptide fragment for use as a medicament in the prevention or treatment of a cancer.

In a further aspect the invention relates to a pharmaceutical composition comprising the CD44ex9-binding protein of the invention or a labelled CD44ex9-binding protein, which are bound to a nanoparticle, and a pharmaceutically acceptable carrier.

The pharmaceutical compositions prepared according to the invention comprise the conjugated nanoparticle together with a pharmaceutically acceptable carrier. The term "pharmaceutically acceptable" includes any carrier which does not interfere with the effectiveness of the biological activity of the CD44ex9-binding protein and which is not toxic to the host to which it is administered.

As one embodiment of the invention, the compositions are prepared using CD44ex9-binding protein stabilized with human serum albumin. For this purpose, a preparation of CD44ex9-binding protein is lyophilised with human serum albumin in vials.

In certain embodiments, the pharmaceutical composition further comprises one or more other drugs that act as toxin, cytokine, cytostatic agent, or immunomodulatory agent.

As used herein, the language "pharmaceutically acceptable carrier" is intended to include any and all solvents, solubilizers, fillers, stabilizers, binders, absorbents, bases, buffering agents, lubricants, controlled release vehicles, diluents, emulsifying agents, humectants, lubricants, dispersion media, coatings, antibacterial or antifungal agents, isotonic and absorption delaying agents, and the like, compatible with pharmaceutical administration. The use of such media and agents for pharmaceutically active substances is well-known in the art. Except insofar as any conventional media or agent is incompatible with the active compound, use thereof in the compositions is contemplated. Supplementary agents can also be incorporated into the compositions.

A pharmaceutical composition of the invention is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils or other synthetic solvents; antibacterial agents such as benzyl alcohol or methyl paraben; antioxidants such as ascorbic acid or sodium bisulfate; chelating agents such as EDTA; buffers such as acetates, citrates or phosphates and agents for the adjustment of tonicity such as sodium chloride or dextrose. The pH value can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions or dispersions and sterile powders for the extemporaneous preparation of sterile injectable solutions or dispersion. For intravenous administration, suitable carriers include physiological saline, bacteriostatic water, Cremophor EL™ (BASF, Ludwigshafen, BRD) or phosphate buffered saline (PBS). In all cases, the injectable composition should be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, and the like), and suitable mixtures thereof. The proper fluidity can be maintained, for example, by the use of a coating such as lecithin, by the maintenance of the required particle size in the case of dispersion and by the use of surfactants.

Prevention of the action of microorganisms can be achieved by various antibacterial and antifungal agents, for example, parabens, chlorobutanol, phenol, ascorbic acid, thiomersal, and the like. In many cases, it will be preferable to include isotonic agents, for example, sugars, polyalcohols such as mannitol, sorbitol, or sodium chloride in the composition. Prolonged absorption of the injectable compositions can be brought about by including in the composition an agent which delays absorption, for example, aluminium monostearate.

Sterile injectable solutions can be prepared by incorporating the active compound (e.g., the conjugated nanoparticle) in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. They can be enclosed in gelatine capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose; an excipient such as starch or lactose; a disintegrating agent such as alginic acid, sodium starch glycollate (e.g. Primogel ®), or corn starch; a lubricant such as magnesium stearate; a glidant such as colloidal silicon dioxide; a sweetening agent such as sucrose or saccharin; or a flavouring agent such as peppermint, methyl salicylate, or orange flavouring.

For administration by inhalation, the compounds are delivered in the form of an aerosol spray from a pressured container or dispenser which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the bioactive compounds are formulated into ointments, salves, or creams as generally known in the art.

The composition can also be prepared in the form of suppositories (e.g., with conventional suppository bases such as cocoa butter and other glycerides) or retention enemas for rectal delivery.

In one embodiment, the therapeutic moieties, which may contain the CD44ex9-binding protein, are prepared with carriers that will protect the compound against rapid elimination from the body, such as a controlled release formulation, including implants and microencapsulated delivery systems. Methods for preparation of such formulations will be apparent to those skilled in the art. The materials can also be obtained commercially from e.g. Alza Corporation and Nova Pharmaceuticals Incorporation. Liposomal suspensions (including liposomes targeted to cancer cells with antibodies to tumor antigens) can also be used as pharmaceutically acceptable carriers. These can be prepared according to methods known to those skilled in the art.

It is especially advantageous to formulate oral or parenteral compositions in dosage unit form for ease of administration and uniformity of dosage. Dosage unit form, as used herein, includes physically discrete units suited as unitary dosages for the subject to be treated; each unit contains a predetermined quantity of active protein calculated to produce the desired therapeutic effect in association with the required pharmaceutical carrier. The specification for the dosage unit forms of the invention are dictated by and directly dependent on the unique characteristics of the active protein and the particular therapeutic effect to be achieved, and the limitations inherent in the art of compounding such an active protein for the treatment of individuals.

Toxicity and therapeutic efficacy of the protein of the invention can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Proteins which exhibit large therapeutic indices are preferred. While proteins that exhibit toxic side effects may be used, care should be taken to design a delivery system that targets such proteins to the site of affected tissue in order to minimize potential damage to non-cancer cells and, thereby, reduce side effects.

The data obtained from the cell culture assays and animal studies can be used in formulating a range of dosage for use in humans. The dosage of such compounds lies preferably within a range of circulating concentrations that includes the ED₅₀ with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC₅₀ (f.e., the concentration of the test compound which achieves a half maximal inhibition of symptoms) as determined in cell culture. Such information can be used to more accurately determine useful doses in humans. Levels in plasma may be measured, for example, by high performance liquid chromatography.

The pharmaceutical compositions can be included in a container, pack, or dispenser together with instructions for administration.

In another aspect, the invention provides a medicament/dosimeter combination package comprising: a) a medicament to be individually dosed, and b) a diagnostic indicator system for a patient-specific property that is relevant for the action, side effect, interaction, metabolism, absorption, distribution, metabolism, and elimination of the medicament to be administered to a patient, wherein the patient-specific property is selected from the group consisting of an endogenous substance, a regulation mechanism, a gene or an indication system. In the above described combination package, the medicament or the diagnostic indicator system can be the isolated CD44ex9-binding protein or any fusion protein or conjugate therewith. For the above described purpose, also the CD44v5 peptides of the invention can be used as a medicament or diagnostic indicator system.

A suitable combination package is disclosed by EP 1 542 644 B1 (MCS Micro Carrier Systems GmbH) together with methods of preparing and using said medicament/dosimeter combination.

Another aspect of invention includes methods for preparing pharmaceutical compositions for modulating the expression or activity of the protein of the present invention. Such methods comprise formulating a pharmaceutically acceptable carrier with an agent which modulates expression or activity of the protein of the present invention. Such compositions can further include additional active agents. Thus, the invention further includes methods for preparing a pharmaceutical composition by formulating a pharmaceutically acceptable carrier with an agent which modulates expression or activity of the protein of the present invention and one or more additional bioactive agents.

### Definitions

In the context of the present invention, the term "CD44ex9-binding protein" is used for a protein with a length of 300 amino acids or less that binds to the polypeptide encoded by exon 9 of the human CD44 gene. In the description it is also referred to as "binding protein" or "active compound".

A protein in the context of the invention is defined as a molecule that is composed of amino acids. The term protein therefore includes dipeptides, tripeptides, tetrapeptides pentapeptides, hexapeptides, heptapeptides, octapeptides, longer oligopeptides and amino acid chains. The amino acid chain can be in a linear or a branched form.

Amino acids in the context of the present invention are organic compounds that contain at least one amino and at least one carboxyl group which in the protein form an amide bond.

The amino acids that are part of the protein can be taken from the 20 standard alpha-amino acids or non-standard amino acids, like selenocysteine, pyrrolysine, lanthionine, hydroxyproline, carboxygluatamate, 2-aminoisobutyric acid, dehydroalanine. Furthermore, other amino acids like beta-amino acids (f.e. beta alanine), gamma amino acids or even higher amino acids could be part of the protein.

For certain amino acids different enantiomeric or stereoisomeric form could be chosen, like e.g. for the alpha amino acids that exist as two enantiomers, the D form and the L-form.

The protein may be manufactured synthetically (by means of organic synthesis in a cell-free system that makes use of a ribosome system that may or may not be artificial) or it may be manufactured naturally by cells that may or may not be genetically manipulated, or by a single cell organism (e.g. by a bacterium or a yeast cell) or a multicellular organism.

The protein could be modified in many ways. Typical modifications are post-translational modifications that include lipids, acetate groups, phosphate groups, or carbohydrates. The protein could also chemically modified with e.g. biotin groups.

The protein can be a multimer consisting of identical subunits (homomer) or different proteins (heteromer) which are hold together by covalent bonds or non-covalent interactions.

The term "binding affinity" used herein refers to the strength of the affinity between the CD44ex9 binding protein and CD44v5 domain and is described by the dissociation constant K_{D}. The K_{D} value for the binding affinity between the CD44ex9 binding protein and CD44v5 domain may be determined by equilibrium methods, (e.g. enzyme-linked immunoabsorbent assay (ELISA) or radioimmunoassay (RIA)) or kinetics (e.g. BIACORE™ analysis), for example.

"K_{D}" refers to the relative binding affinity between the CD44ex9 binding protein and CD44v5 domain. High K_{D} values represent low binding affinity.

In the context of the invention the term "acute treatment" refers to a short-term medical treatment, usually in a hospital, for patients having an acute illness or injury or recovering from surgery. The term "post-acute treatment" refers to a mid-term medical treatment, usually in a hospital, for patients having an acute illness or injury or recovering from surgery.

The term "treating" or "treatment" refers to any medical measure for preventing, reducing, mitigating or curing physiological disorders within a mammal, in particular a human, in the need thereof. According the invention the treatment also encompasses a targeted drug delivery.

A "therapeutically effect amount" is defined as the amount of active ingredient that will reduce the symptoms associated with a neurological or neurodegenerative disease, such as stroke. "Therapeutically effective" also refers to any improvement in disorder severity or the frequency of incidences compared to no treatment. The term "treatment" encompasses either curing or healing as well as mitigation, remission or prevention.

The term "expression vector" refers to vectors that have the ability to incorporate and express heterologous DNA fragments in a foreign cell. Many prokaryotic and eukaryotic expression vectors are known and/or commercially available. Selection of appropriate expression vectors is within the knowledge of the skilled person.

In the context of the invention the term "nanoparticle" refers to a particle with a size below 1 µm and preferably between 1 and 100 nm. Thus, the term "nanoparticle" includes nanocrystals made from semiconductor materials (so called quantum dots), as well as nanoparticles consisting of a Noble metal cluster, rare-earth based inorganic luminescent nanoparticles, superparamagnetic iron oxide nanoparticles (IONPs), block-copolymer micelles, nanocells, dendrimers, nanotubes, polymersomes, XPclad® nanoparticles, and nanoparticles consisting of amorphous silica surrounded by a crystalline luminescent calcium phosphate layer (e.g. ORMOBEAD ®).

### Literature

Dembski S, Probst J, Kockenbring T and Barth S, News Analytik, 18.1.2013; p. 1 -3.
Dembski S, Rupp S, Milde M, Gellermann C, Dyrba M, Schweizer S, Batentschuk M, Osvet A, Winnacker A, Optical Materials, 2011, p.1106-1110. (2011 a)
Dembski S, Gellermann C, Probst J, Klockenbring T, Barth S, GIT-Labor-Fachzeitschrift 01/2011: 48-49. (2011 b)
Deonarain MP, Kousparou CA, Epenetos AA, MAbs, 2009, 1(1): 12-25.
Goding JW, Monoclonal Antibodies: Principles & Practice (Academic Press, London, 2nd edition, 1986), p. 61-63.
Günthert U, Hofmann M, Rudy W, Reber S, Zöller M, L Haubmann L, Manzku S, Wenzel A, Ponta A, Herrlich P: "A new variant of glycoprotein CD44 confers metastatic potential to rat carcinoma cells", Cell 1991, 65: 13-24.
Hale LP and Haynes BF, J Immunol. 1992, 149(12): 3809-3816.
Jalkanen ST, Bargatze RF, Herron LR, Butcher EC. A lymphoid cell surface glycoprotein involved in endothelial cell recognition and lymphocyte homing in man. Eur J Immunol. 1986 Oct;16(10):1195-1202.
Johnson P & Ruffell B, Inflamm Allergy Drug Targets, 2009, 8(3): 208 - 220.
Kampmeier F, Ribbert M, Nachreiner T, Dembski S, Beaufils F, Brecht A and Brecht F, Bioconjugate Chem 2009, 20(5): 1010 - 1015.
La Fleur L, Johansson A-C, Roberg K, PLoS ONE 2012, 7(9): e44071
Nagano O and Saya H, Cancer Sci 2004, 95(12): 930-935.
Piotrowicz RS, Damaj BB, Hachicha M, Incardona F, Howell SB and Finlayson M, Mol Cancer Ther 2011, 10(11): 2072 - 2082.
Ponta H, Sherman L, Herrlich PA, Nat Rev Mol Cell Biol. 2003, 4(1): 33-45.
Probst J, Dembski S, Milde M, Rupp S, Expert Review of Molecular Diagnostics 2012, 12(1): 49-64.
Seiter BS, Arch R, Reber S, Komitowski D, Hofmann M, Ponta H, Herrlich P, Matzku S, and Zoller M. Prevention of tumor metastasis formation by anti-variant CD44. J Exp Med, 177: 443-455, 1993.
Saturag et al 2000; British Journal of Nutrition; 2000, (84), 791-802
Singh R and Lillard Jr. JW, Exp Mol Pathol, 2009, 86(3): 215-223.
Tempfer C, Lösch A, Heinzl H, Häusler G, Hanzal E, Kölbl H, Breitenecker G, Kainz C, Eur J Cancer, 1996, 32A: 2023-2025.
Vizoso FJ, Fernandez JC, Corte MD, Bongera M, Gava R, Allende MT, Garcia-Muniz JL, Garcia-Moran M, J Cancer Res Clin Oncol, 2004, 130: 679-686.
Xie S, Lee S, Chen X, Adv Drug Deliv Rev 2010, 62(11): 1064-1079.
Zhang S, Wu CC, Fecteau JF, Cui B, Chen L, Zhang L, Wu R, Rassenti L, Lao F, Weigand S, Kipps TJ, Proc Natl Acad Sci USA, 2013, 110: 6127-6132.

### Figure legends

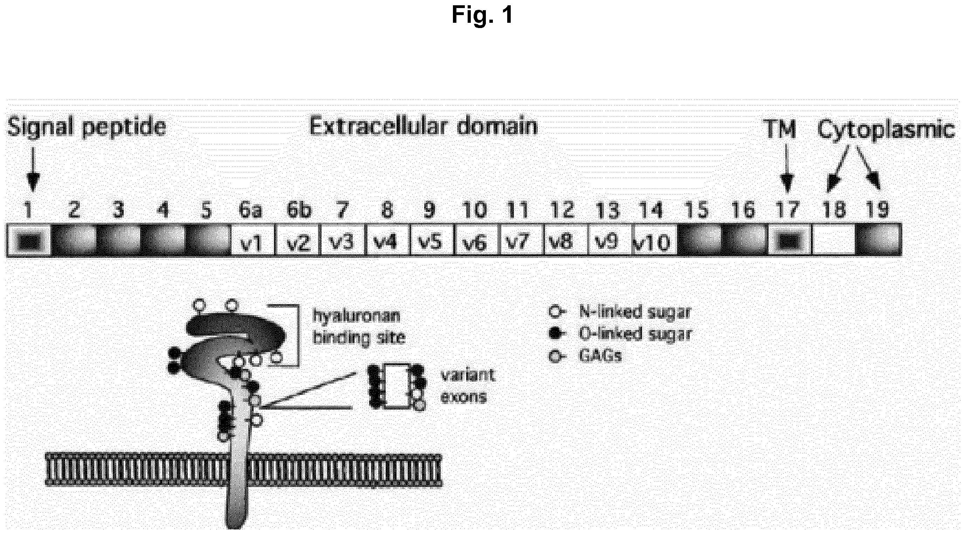

- Fig. 1:: Genomic structure and protein domains of CD44.
- Fig. 2:: (A) Schematic outline of the strategy of the yeast-two-hybrid screening. (B) Vector used for the Y2H-screen.
- Fig. 3:: Amino acid sequences of the positive CD44-interacting clones together with the consensus sequence derived from it.
- Fig. 4:: Labeling of HT29 cells with the nanoparticle conjugate Q_CA19-9-3.
- Fig. 5:: Negative control for labeling of HT29 cells with the nanoparticle conjugate Q_CA19-9-5.
- Fig. 6:: Quantification of HT29 cell-labeling with the nanoparticle conjugate Q_CA19-9-3 or the negative control MBP-nanoparticle.
- Fig. 7:: Competition of HT29 cell-labeling with the nanoparticle conjugate Q_CA19-9-3 by addition of increasing amounts of the free target CA19-9.
- Fig. 8:: Labeling of Colo29 cells with the nanoparticle conjugate Q_CA19-9-3.
- Fig. 9:: Negative control for labeling of Colo29 cells with the nanoparticle conjugate Q_CA19-9-5.
- Fig. 10:: Labeling of SW116 cells with the nanoparticle conjugate Q_CA19-9-3.
- Fig. 11:: Negative control for labeling of SW116 cells with the nanoparticle conjugate Q_CA19-9-5.
- Fig. 12:: Labelling of mucinous adenocarcinoma tissue (sample No. 35) with a bispecific nanoparticle Q_CEA/CA19-9_12 conjugated to an anti-CEA and an anti-CA-19-9 antibody. In the four quadrants, the following conditions were analysed:
Upper left: Labeling with Q_CEA/CA19-9_12
Upper right: Labeling with CEA/CA19-9_12 by addition of the free target MBP-CEA (25 µM)
Lower left: Labeling with Q_CEA/CA19-9_12 by addition of the free target Sialyl LewiSₐ (250 µM)
Lower right: Labeling with Q_CEA/CA19-9_12 by addition of the free targets MBP-CEA (25 µM) and Sialyl Lewis a (250 µM)

### EXAMPLES

### Example 1: Isolation of CD44ex9-binding proteins

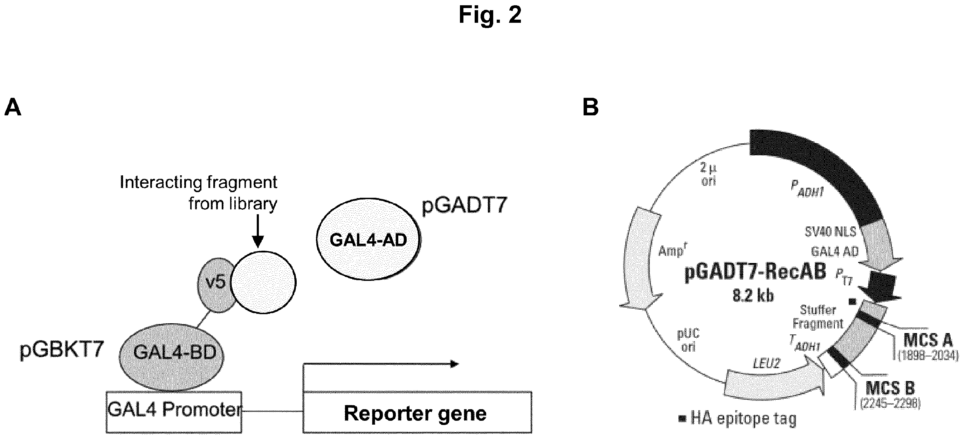

For the isolation of proteins or protein fragments binding to the v5 domain of CD44 a competitive two hybrid screening was performed according to the method disclosed in EP 1 721 974 A1. As bait, the v5-fragment was fused the GAL4 binding domain. The respective ORF was cloned into a plasmid vector and designated as pGBKT7 (see Figure 2). A human cDNA library with fragments fused to the GAL4-activation domain was used as a prey (vector pGADT7-RecAB; see Figure 2).

The screening was performed under stringent selection conditions and resulted in the identification of 39 positive clones. After selective co-transformation of the bait and the pray plasmid, 21 of the 39 clones remained positive. The 21 clones were sequenced and analysed by BLAST search. As result the 21 sequences could be reduced to 12 different sequences since four sequences were identified twofold and one sequence was present in four clones.

Furthermore, a sequence comparison revealed that these five sequence families show extensive sequence homologies allowing the formation of a consensus sequence (see Figure 3).

### Example 2: In vitro detection of antigen-expressing tumor cells

### 2.1 Background and Objective

In order to demonstrate the ability of the protein-quantum dots conjugates to specifically detect antigen-expressing tumor cells, a tumor specimen or in vitro cultivated tumor cells were analysed by fluorescence and in parallel by immunohistochemistry for expression of the respective antigen. As a first antigen, the carcinoembryonic antigen (CEA) was analysed. CEA is a glycoprotein involved in cell adhesion. As a detection-ligand, the anti CEA antibody was conjugated to the quantum dot QDBP-655 (charge #773780). Furthermore, the cancer antigen 19-9 (CA19-9) was analysed. CA19-9 is a sialylated Lewis (a) antigen and represents a tumor marker that is used primarily in the management of pancreatic cancer.

### 2.2. Samples

### 2.2.1 Cell culture samples

In a first set of experiments, the in vitro cultivated human cancer cell lines HT29, Colo25 and SW116 were used. HT29 is an adherent colorectal adenocarcinoma cell line, Colo25 a human colon carcinoma cell line and SW116 a colorectal cancer cell line.

### 2.2.2. Tumor sample

In a separate set of experiments, a sample taken from a human tumor was used. The tumor sample No. 35 was resected in 2011 from coecum and was diagnosed as a mucinous adenocarcinoma. It was classified according the TNM classification as pT4No(0/18)G3Ro. This sample which was not otherwise pretreated was prepared and fixed as follows.

### Preparation and fixation of tissue sections

The resected tumor sample, stored at -70°C was used to prepare cryosections with a thickness of 5 µm using a HM560 MV cryostate (Thermo Scientific, Walldorf, Germany). The sections were dried for 60 to 120 min at room temperature and incubated with cold acetone (-20°C) for 10 minutes. After a further drying step for 10 min at room temperature, the sections were stored over night at -70°C.

The sections were defrosted in a closed box for 30 min at room temperature. Fixation was performed by incubation in a solution containing 20 mg/ml dimethyl suberimidate (DMS) and 20 mM CaCl₂ in 250 mM Tris-HCl buffer pH 8.0. Afterwards the sections were washed for 5 minutes in D-PBS-T buffer containing 130 mM NaCl, 7 mM Na₂HPO₄, 3 mM KH₂PO₄ and 0.1% Tween 20 by a quenching step for 20 minutes in a buffer containing 130 mM NaCl, 7 mM Na₂HPO₄, 3 mM KH₂PO₄ and 200 mM glycerine. In a final step the sections were washed for 5 minutes in D-PBS-T buffer.

### Counterstaining of the tissue sections.

Counterstaining was performed using the Dako Autostainer Plus (DAKO Deutschland GmbH, Hamburg, Germany) according the following protocol:
- Rinsing with D-PBS-T buffer
- Blocking for 60 min with 1% BSA / 5% goat serum in D-PBS buffer
- Blowing off
- Incubation with 2 x 100 µl conjugate (20nM in 1% BSA / 5% goat serum in D-PBS buffer) for 60 min
- Rinsing for three times with D-PBS-T
- Blowing off
- Incubation with 2 x 100 µl primary antibody (20nM in 1% BSA / 5% goat serum in D-PBS buffer) for 60 min
- Rinsing for three times with D-PBS-T
- Blowing off
- Incubation with 2 x 100 µl secondary antibody (20nM in 1% BSA / 5% goat serum in D-PBS buffer) for 60 min
- Rinsing for three times with D-PBS-T
- Blowing off
- Incubation with 3 x 200 µl Hoechst 33342 (2µg/ml in D-PBS buffer) for 10 min
- Rinsing for three times with D-PBS-T
- Embedding in Mowiol/Triethylenediamine (DABCO)

### Microscopic Evaluation

The sections as prepared above were evaluated with a reverse microscope Axiovert 200 with Axiocam HrM and a Axiocam Hrc CCD camera system, HXP 120 C illuminating device. The pictures were analysed using the Axiovision software (version 4.8; Carl Zeiss, Oberkochen, Germany).

### 2.3 Protein-quantum dot conjugates

For antigen detection the quantum dots as listed in the following table were prepared:

| Conjugate | Type of QDBP-655 quantum dot | Ligand | QDBP/Ligand ratio |
|---|---|---|---|
| Q561 | #773780 | SI6166 | N/A |
| Q_CA19-9-20 | #773780 | MBP-NS-19-9-scFv-His₆ + SI6166 | 1:50 |
| Q_CEA/CA19-9_9 | Q561 | MBP-NS-19-9-scFv-His₆ + SI6166 | 1:10 |
| Q_CEA/CA19-9_10 | Q561 | MBP-NS-19-9-scFv-His₆ + SI6166 | 1:20 |
| Q_CEA/CA19-9_11 | Q561 | MBP-NS-19-9-scFv-His₆ + SI6166 | 1:30 |
| Q_CEA/CA19-9_12 | Q561 | MBP-NS-19-9-scFv-His₆ + SI6166 | 1:40 |
| Q_CEA/CA19-9_13 | Q561 | MBP-NS-19-9-scFv-His₆ + SI6166 | 1:50 |
| Q_CEA/CA19-9_1 | Q561 | MBP-NS-19-9-scFv-His₆ + SI6166 | 1:50 |

The conjugate Q561 is a conjugate between the quantum dot QDBP-655 (batch #773780) and the ligand SI6166. QDBP-655 has a hydrodynamic diameter of 6 nm and possesses a Ni-NTA ligand. The ligand SI6166 is an CEA-binding protein having a hydrodynamic diameter of 2.1 nm, and the final conjugate exhibits a hydrodynamic diameter of 10.1 nm. The anti CEA protein is coupled to the quantum dots.

The quantum dots Q_CA19-9-x are conjugates between the quantum dot QDBP-655 (batch #773780) and the anti-CA 19-9 antibody "MBP-NS-19-9-scFv-His6". This anti-CA19-9 antibody which is a recombinant antibody mimetic consisting of the variable region of the heavy chain (VH) and the light chain (VL) and can be described as a "single chain fragment of variable regions", scFv. This scFv fragment is fused to a His-tag which is used for the coupling to the quantum dot. The anti-CA 19-9 antibody "MBP-NS-19-9-scFv-His6" is also denominated as SI6950.

The quantum dots Q_CEA/CA19-9_x are bispecific conjugates, whereby the quantum dot Q561 harboring the anti CEA-antibody SI6166 is further conjugated with the anti-CA 19-9 antibody SI6950.

Different batches of the conjugate Q_CEA/CA19-9_x were prepared differing by the ratio of the quantum dot to the ligand (see last column of the table).

### 3. Results of CA19-9 conjugated nanoparticles

### 3.1 HT29 cells

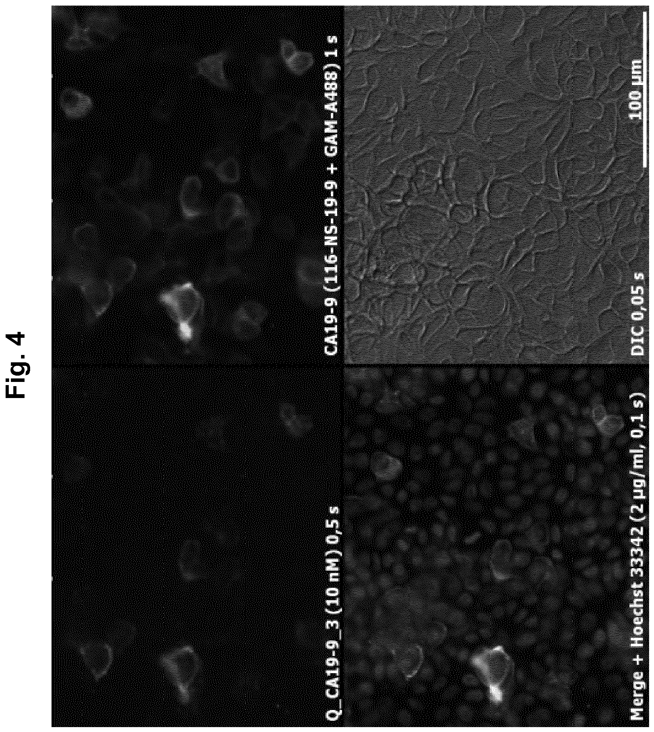

As shown in upper left panel of Fig. 4, the conjugate Q_CA19-9-3 labels the cell membrane of the HT29 cells. A staining of the cells with the anti-CA19-9 antibody detected by Alexa Fluor 488 fluorescence (upper right panel) shows a perfect co-localization of the signals showing the specificity of the conjugate binding. In the lower right panel the cells are shown by differential interference contrast (DIC) microscopy.

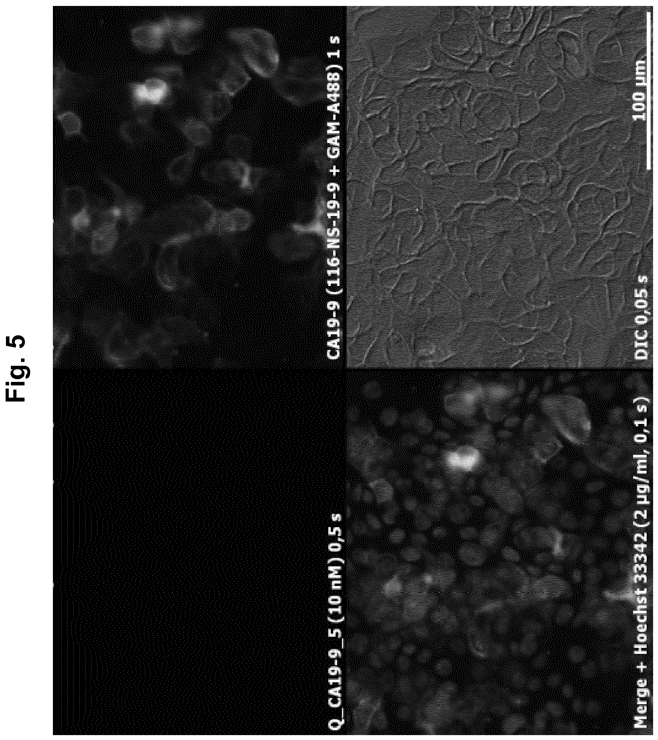

In the negative control a quantum dot conjugated to MBP (MBP-His/QDP655) was used. As shown in the upper left panel of Fig. 5, no labeling could be detected. The presence of the CA19-9 antigen was verified by staining of the cells with the anti-CA19-9 antibody detected by Alexa Fluor 488 fluorescence (upper right panel).

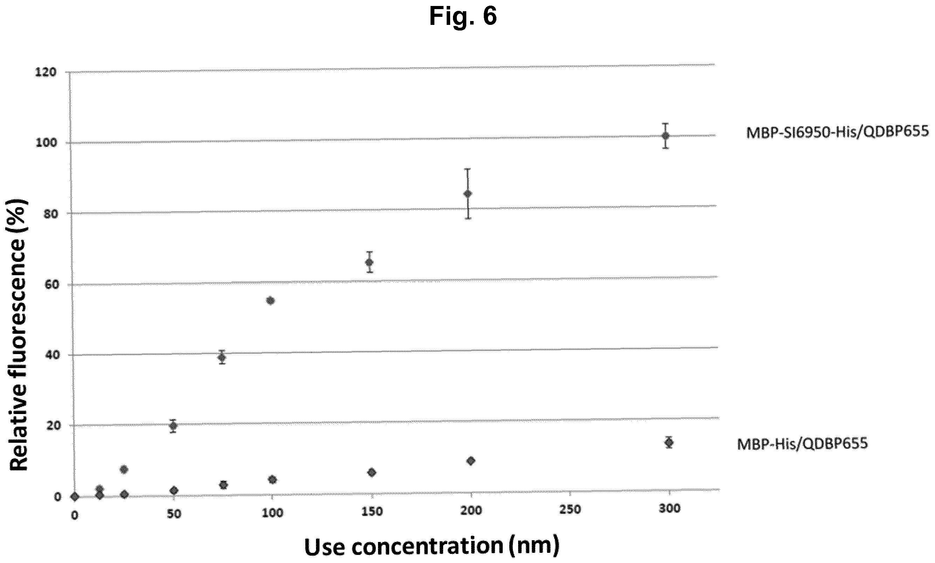

The specific labeling of the CA19-9 antigen was also shown by a titration experiment. A shown in Fig. 6, an increasing amount of the conjugate Q_CA19-9_3 leads to an increased labeling of the HT29 cells as demonstrated by increasing relative fluorescence. In contrast, the negative control MBP-His/QDP655 leads only to a sparse fluorescent labeling which show no major increase at higher conjugate concentrations.

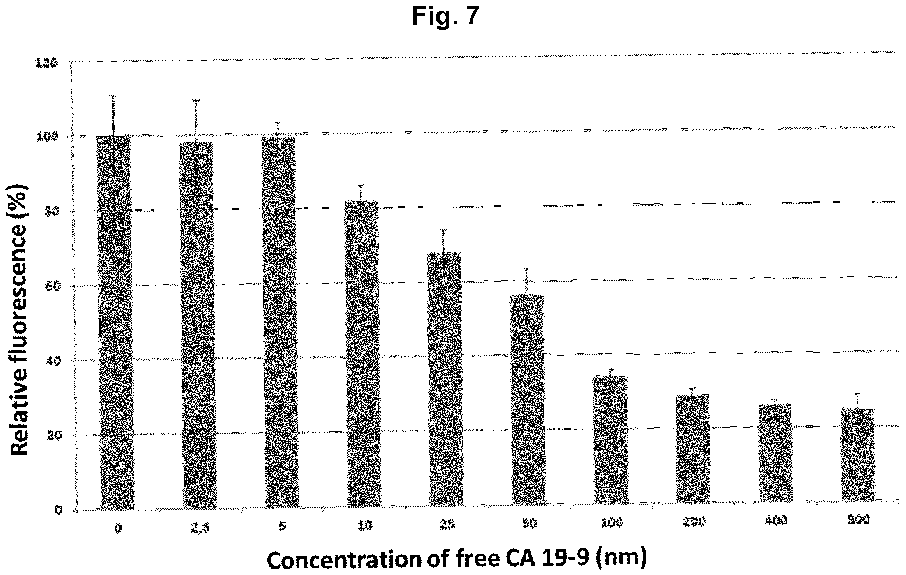

The specificity of the CA19-9 labeling was further verified by a competition experiment as shown in Figure 7: An increasing amount of free CA19-9 antigen added to the labeling mixture leads to a decreased CA19-9 labeling.

### 3.2 Colo29 cells

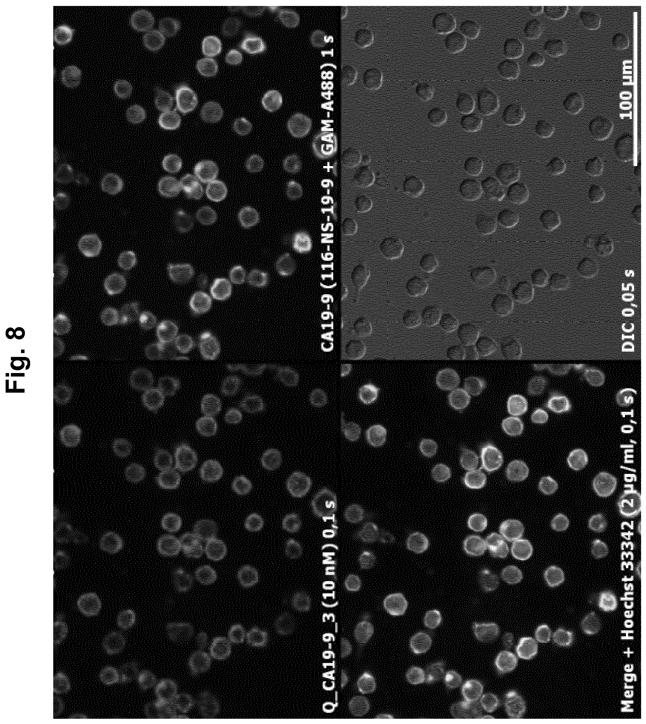

As shown in upper left panel of Fig. 8, the conjugate Q_CA19-9-3 labels the cell membrane of the Colo29 cells. A staining of the cells with the anti-CA19-9 antibody detected by Alexa Fluor 488 fluorescence (upper right panel) shows a perfect colocalization of the signals showing the specificity of the conjugate binding. In the lower right panel the cells are shown by differential interference contrast (DIC) microscopy.

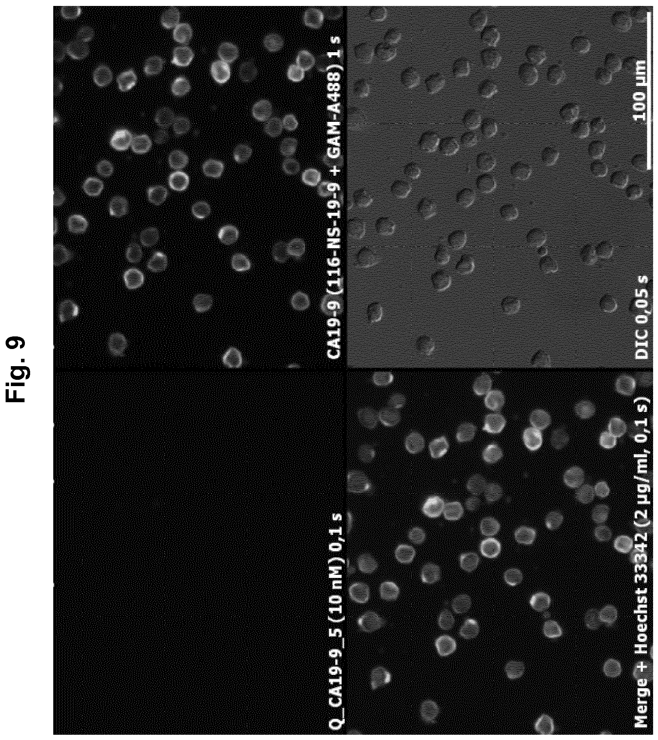

In the negative control a quantum dot conjugated to MBP (MBP-His/QDP655) was used. As shown in the upper left panel of Fig. 9, no labeling could be detected. The presence of the CA19-9 antigen was verified by staining of the cells with the anti-CA19-9 antibody detected by Alexa Fluor 488 fluorescence (upper right panel).

### 3.3 SW116 cells

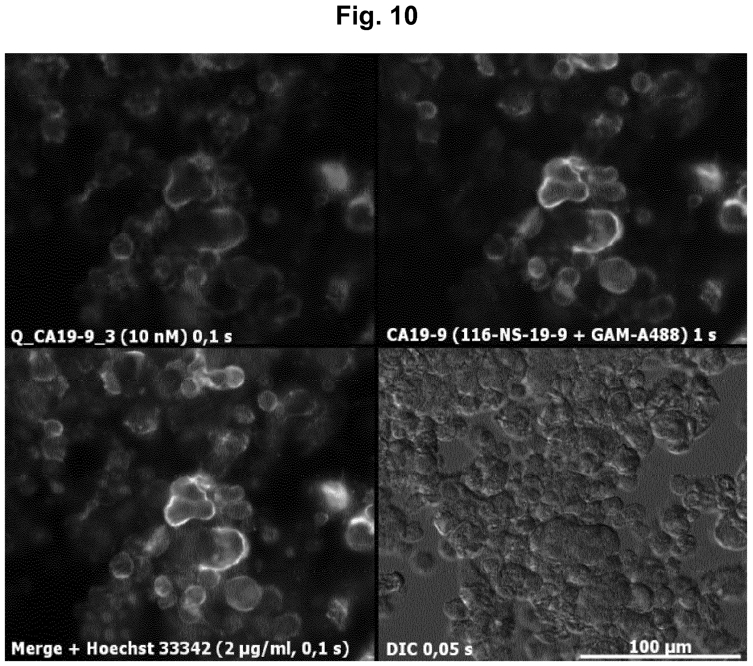

As shown in upper left panel of Fig. 10, the conjugate Q_CA19-9-3 labels the cell membrane of the Colo29 cells. A staining of the cells with the anti-CA19-9 antibody detected by Alexa Fluor 488 fluorescence (upper right panel) shows a perfect colocalization of the signals showing the specificity of the conjugate binding. In the lower right panel the cells are shown by differential interference contrast (DIC) microscopy.

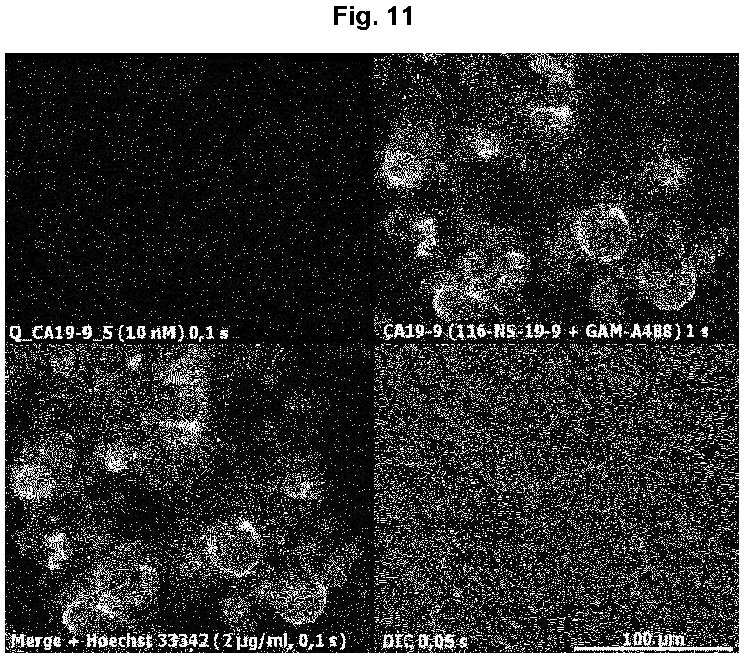

In the negative control a quantum dot conjugated to MBP (MBP-His/QDP655) was used. As shown in the upper left panel of Fig.11, no labeling could be detected. The presence of the CA19-9 antigen was verified by staining of the cells with the anti-CA19-9 antibody detected by Alexa Fluor 488 fluorescence (upper right panel).

### 4. Results of bispecific conjugated nanoparticles

This experiment was performed in order to test the ability of the bispecific conjugated nanoparticle to interact with both antigens in a given target. For this purpose the nanoparticle conjugated with both the anti-CA19-9 antibody and the anti CEA antibody was used to detect the respective antigens on the tumor cells of above described sample No. 35. The specificity was demonstrated by inhibition with the free targets MBP-CEA and 19-9.

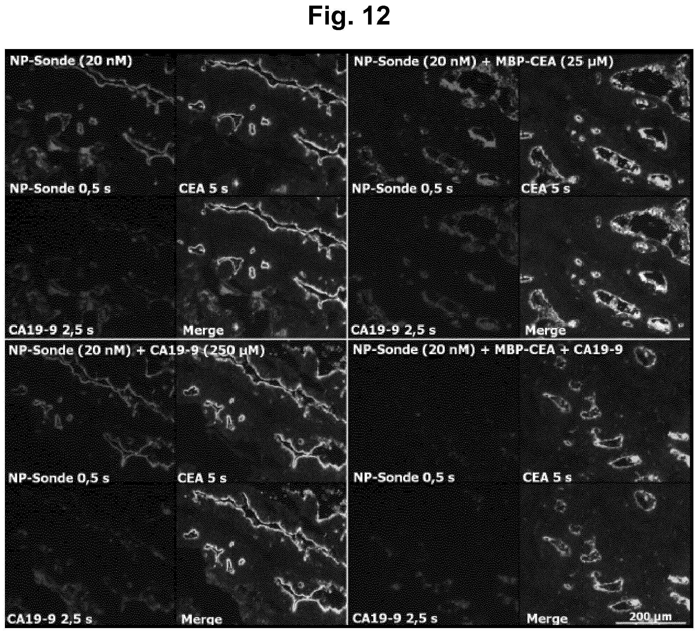

As shown in the upper left part of Figure 12, the bispecific nanoparticles labeled cell membranes of the tumor tissue. A staining of the tumor tissue with an anti-CA19-9 antibody or an anti CEA-antibody showed in each case a partial colocolization with combines to the complete labeling profile of the nanoparticle,

When the bispecific nanoparticle is used in combination with the free target MBP-CEA, a labeling can be observed that shows a perfect colocalization with the staining of the anti-CA-19-9 antibody (see Figure 12, upper right quadrant). Hence, the free target inhibits the interaction with the cellular-bound CEA but still allows a labeling of the CA19-9.

As shown in the lower left quadrant of Figure 12, the addition of the free target Sialyl-Lewisₐ inhibits the interaction with the cellular CA19-9 antigen but retains the CEA-labeling.

In a final experiment the bispecific nanoparticles were combined with both free targets, the MBP-CEA and Sialyl-Lewisₐ (see lower right quadrant of Figure 12). As a result the labeling was completely inhibited (upper left corner of the quadrant). The colocalized expression of both antigens was verified by staining with the respective anti-CEA and CA19-9 antibodies.

## Claims

1. A nanoparticle conjugated to a protein capable of binding to a polypeptide encoded by exon 9 of human CD44 (CD44ex9), said protein comprises or consists of
(i) an amino acid sequence according SEQ ID No. 1 to 5; or
(ii) an amino acid sequence with at least 95% identity to the amino acid sequence given in SEQ ID No. 1 to 5 as a whole,
wherein said protein has a length of 100 amino acids or less.

2. The conjugated nanoparticle according to claim 1, wherein the CD44ex9-binding protein binds to a CD44 isoform comprising the domain encoded by exon 9.

3. The conjugated nanoparticle according to claim 2, wherein the CD44 isoform is selected from the list consisting of:
a) CD44 v5,
b) CD44 v5 -v6,
c) CD44 v3 - v6,
d) CD44 v3 - v6,
e) CD44 v2 - v10,
f) CD44 v3 - v10,
g) CD44 v4 - v7,
h) CD44 v4 - v10,

4. The conjugated nanoparticle according to claims 1 to 3 further conjugated to at least one tumor antigen-binding substance and/or cytotoxic agent.

5. The conjugated nanoparticle according claim 4, wherein said tumor antigen-binding substance is selected from the list consisting of an antibody against CEA, an antibody against CA-19-9, an adhesin, which is preferably modified, and combinations thereof.

6. The conjugated nanoparticle according claims 1 to 5, wherein the nanoparticle is selected from the group consisting of quantum dots, noble metal clusters, superparamagnetic iron oxide nanoparticles (IONPs), block-copolymer micelles, nanocells, dendrimers, nanotubes, polymersomes, XPclad® nanoparticles, nanoparticles consisting of amorphous silica surrounded by a crystalline luminescent calcium phosphate layer, Sio₂/Zn₂SiO₄:Mn²⁺ and SiO₂/Ca₁₀(PO₄)₆OH:Eu³⁺ core-shell nanoparticles with diameters below 100 nm and luminescent dye labeled hybrid nanoparticles.

7. The conjugated nanoparticle according claims 1 to 5, wherein the nanoparticle has an inorganic core and a coating layer comprising an imidazole component, with the smallest diameter of the inorganic core including the coating layer being no more than 15 nm.

8. The conjugated nanoparticle according claim 7, wherein the imidazole component is a peptide comprising at least one histidyl residue, and preferably a dipeptide.

9. The conjugated nanoparticle according claim 8, wherein the imidazole component is a mixture of different histidyl-containing dipeptides.

10. The conjugated nanoparticle as claimed in any one of claims 1 to 9, wherein the nanoparticle is non-inert.

11. The conjugated nanoparticle as claimed in any of claims 1 to 10 for the use in the in vivo diagnosis or treatment of a disease selected from the group consisting of autoimmune diseases such as insulin-dependent diabetes, multiple sclerosis, Sjögren's syndrome or systemic lupus erythematosus (SLE); skin diseases as psoriasis or atopic dermatitis; chronic inflammatory diseases such as rheumatoid arthritis or inflammatory bowel disease; tissue injury; allergic diseases and cancer disease.

12. The conjugated nanoparticle according to claims 1 to 10, for the in vivo use for distinguishing abnormal, pre-cancerogenic or cancerogenic tissue from normal tissue or for assisting in visual assessment during surgical intervention...

13. The nanoparticle as claimed in claim 12, wherein the contrast agent is capable of identifying cancer cells or carcinoma in situ cells.

14. The nanoparticle as claimed in claim 13, wherein the cancer cells to be identified are selected from the list consisting of:
a) Adenocarcinoma cells;
b) Thymic epithelial tumor cells;
c) Cervical carcinoma cells;
d) Non-Hodgkin lymphoma cells;
e) Lung cell carcinoma cells;
f) Pancreas carcinoma cells; and
g) Cancer stem cells

15. The use of the conjugated nanoparticle as claimed in any of claims 1 to 10 as an ex vivo or in vitro diagnostic agent for diagnosis of a disease selected from the group consisting of autoimmune diseases such as insulin-dependent diabetes, multiple sclerosis, Sjögren's syndrome or systemic lupus erythematosus (SLE); skin diseases as psoriasis or atopic dermatitis; chronic inflammatory diseases such as rheumatoid arthritis or inflammatory bowel disease; tissue injury; allergic diseases and cancer disease.

16. A pharmaceutical composition comprising the conjugated nanoparticle as claimed in any one of claims 1 to 10 and a pharmaceutically acceptable excipient.

17. A medicament/dosimeter combination package comprising:
a) a medicament to be individually dosed, and
b) a diagnostic indicator system for a patient-specific property that is relevant for the action, side effect, interaction, metabolism, absorption, distribution, metabolism, and elimination of the medicament to be administered to a patient,
wherein the patient-specific property is selected from the group consisting of an endogenous substance, a regulation mechanism, a gene or an indication system, and
wherein the medicament or the diagnostic indicator system comprises a conjugated nanoparticle as claimed in any one of claims 1 to 10.

## Patentansprüche

1. Nanopartikel, konjugiert an ein Protein, das an ein von Exon 9 von humanem CD44 (CD44ex9) kodiertes Polypeptid binden kann, wobei das Protein
(i) eine Aminosäuresequenz nach SEQ ID Nr. 1 bis 5 oder
(ii) eine Aminosäuresequenz mit insgesamt mindestens 95 %iger Identität zu der in SEQ ID Nr. 1 bis 5 angegebenen Aminosäuresequenz umfasst oder daraus besteht,
wobei das Protein eine Länge von 100 Aminosäuren oder weniger aufweist.

2. Konjugiertes Nanopartikel nach Anspruch 1, wobei das CD44ex9-bindende Protein an eine CD44-Isoform bindet, die die von Exon 9 kodierte Domäne umfasst.

3. Konjugiertes Nanopartikel nach Anspruch 2, wobei die CD44-Isoform aus der Liste ausgewählt ist, die aus:
a) CD44 v5,
b) CD44 v5 - v6,
c) CD44 v3 - v6,
d) CD44 v3 - v6
e) CD44 v2 - v10
f) CD44 v3 - v10,
g) CD44 V4 - v7,
h) CD44 v4 - v10,
besteht.

4. Konjugiertes Nanopartikel nach einem der Ansprüche 1 bis 3, darüber hinaus konjugiert an mindestens eine Tumorantigen-bindende Substanz und/oder zytotoxischen Wirkstoff.

5. Konjugiertes Nanopartikel nach Anspruch 4, wobei die Tumorantigen-bindende Substanz ausgewählt ist aus der Liste bestehend aus einem Antikörper gegen CEA, einem Antikörper gegen CA-19-9, einem Adhäsin, das vorzugsweise modifiziert ist, und Kombinationen derselben.

6. Konjugiertes Nanopartikel nach einem der Ansprüche 1 bis 5, wobei das Nanopartikel ausgewählt ist aus der Gruppe bestehend aus Quantenpunkten, Edelmetallclustern, superparamagnetischen Eisenoxid-Nanopartikeln (IONPs), Block-Copolymer-Mizellen, Nanozellen, Dendrimeren, Nanoröhren, Polymersomen, XPclad®-Nanopartikeln, Nanopartikeln bestehend aus amorphem Siliziumdioxid umgeben von einer kristallinen, lumineszenten Kalziumphosphatsschicht, SiO₂/Zn₂SiO₄:Mn²⁺ und SiO₂/Ca₁₀(PO₄)₆OH:Eu³⁺ Core-Shell-Nanopartikeln mit Durchmessern unter 100 nm und mit lumineszentem Farbstoff-markierten Hybrid-Nanopartikeln.

7. Konjugiertes Nanopartikel nach einem der Ansprüche 1 bis 5, wobei das Nanopartikel einen anorganischen Kern und eine Überzugsschicht, umfassend eine Imidazolkomponente, besitzt, wobei der geringste Durchmesser des anorganischen Kerns einschließlich der Überzugsschicht nicht mehr als 15 nm beträgt.

8. Konjugiertes Nanopartikel nach Anspruch 7, wobei es sich bei der Imidazolkomponente um ein Peptid handelt, das mindestens einen Histidylrest und vorzugsweise ein Dipeptid umfasst.

9. Konjugiertes Nanopartikel nach Anspruch 8, wobei es sich bei der Imidazolkomponente um eine Mischung von verschiedenen Histidylenthaltenden Dipeptiden handelt.

10. Konjugiertes Nanopartikel nach einem der Ansprüche 1 bis 9, wobei das Nanopartikel nicht-inert ist.

11. Konjugiertes Nanopartikel nach einem der Ansprüche 1 bis 10 zur Anwendung bei der in-vivo Diagnose oder Behandlung einer Erkrankung ausgewählt aus der Gruppe bestehend aus Autoimmunerkrankungen, wie Insulin-pflichtiger Diabetes, Multiple Sklerose, Sjögren'sches Syndrom oder systemischer Lupus erythematodes (SLE); Hauterkrankungen wie Psoriasis oder atopische Dermatitis; chronisch-entzündliche Erkrankungen wie rheumatoide Arthritis oder entzündliche Darmerkrankungen; Gewebeverletzungen; allergische Erkrankungen und Krebserkrankungen.

12. Konjugiertes Nanopartikel nach einem der Ansprüche 1 bis 10 zur in vivo Anwendung zur Unterscheidung von abnormem, präkanzerogenem oder kanzerogenem Gewebe von normalem Gewebe oder zur Unterstützung bei der visuellen Beurteilung im Rahmen einer chirurgischen Intervention.

13. Nanopartikel nach Anspruch 12, wobei das Kontrastmittel in der Lage ist, Krebszellen oder Karzinoma in situ-Zellen zu identifizieren.

14. Nanopartikel nach Anspruch 13, wobei die zu identifizierenden Krebszellen aus der Liste ausgewählt sind, die aus:
a) Adenokarzinomzellen,
b) Thymusepithel-Tumorzellen
c) Zervixkarzinomzellen,
d) Non-Hodgkin-Lymphomzellen,
e) Lungenzell-Karzinomzellen
f) Bauchspeicheldrüsen-Karzinomzellen und
g) Krebs-Stammzellen
besteht.

15. Konjugiertes Nanopartikel nach einem der Ansprüche 1 bis 10 als ein ex vivo oder in vitro Diagnostikum zur Diagnose einer Erkrankung ausgewählt aus der Gruppe bestehend aus Autoimmunerkrankungen, wie Insulin-pflichtiger Diabetes, Multiple Sklerose, Sjögren'sches Syndrom oder systemischer Lupus erythematodes (SLE); Hauterkrankungen wie Psoriasis oder atopische Dermatitis; chronisch-entzündliche Erkrankungen wie rheumatoide Arthritis oder entzündliche Darmerkrankungen; Gewebeverletzungen; allergische Erkrankungen und Krebserkrankungen.

16. Pharmazeutische Zusammensetzung umfassend das konjugierte Nanopartikel nach einem der Ansprüche 1 bis 10 und einen pharmazeutisch verträglichen Hilfsstoff.

17. Medikament/Dosimeter-Kombinationspackung umfassend:
a) ein individuell zu verabreichendes Medikament, und
b) ein diagnostisches Indikatorsystem für eine patientenspezifische Eigenschaft, die für Wirkung, Nebenwirkung, Wechselwirkung, Stoffwechsel, Resorption, Verteilung, Stoffwechsel und Ausscheidung des bei einem Patienten zu verabreichenden Medikamentes relevant ist,
wobei die patientenspezifische Eigenschaft aus der Gruppe ausgewählt ist, die aus einer endogenen Substanz, einem Regulationsmechanismus, einem Gen oder einem Anzeigesystem besteht, und
wobei das Medikament oder das diagnostische Indikatorsystem ein konjugiertes Nanopartikel nach einem der Ansprüche 1 bis 10 umfasst.

## Revendications

1. Nanoparticule conjuguée à une protéine capable de se fixer à un polypeptide codé par l'exon 9 de CD44 humain (CD44ex9), ladite protéine comprend ou est constituée de :
(i) une séquence d'acides aminés selon SEQ ID N° 1 à 5 ; ou
(ii) une séquence d'acides aminés avec au moins 95 % d'identité à la séquence d'acides aminés donnée dans SEQ ID N° 1 à 5 dans l'ensemble,
dans laquelle ladite protéine a une longueur de 100 acides aminés ou moins.

2. Nanoparticule conjuguée selon la revendication 1, dans laquelle la protéine se fixant à CD44ex9 se fixe à une isoforme de CD44 comprenant le domaine codé par l'exon 9.

3. Nanoparticule conjuguée selon la revendication 2, dans laquelle l'isoforme de CD44 est sélectionnée parmi la liste constituée de :
a) CD44 v5,
b) CD44 v5-v6,
c) CD44 v3-v6,
d) CD44 v3-v6,
e) CD44 v2-v10,
f) CD44 v3-v10,
g) CD44 V4-v7,
h) CD44 v4-v10.

4. Nanoparticule conjuguée selon l'une quelconque des revendications 1 à 3 conjuguée en outre à au moins une substance se fixant à l'antigène tumoral et/ou un agent cytotoxique.

5. Nanoparticule conjuguée selon la revendication 4, dans laquelle ladite substance se fixant à l'antigène tumoral est sélectionnée parmi la liste constituée d'un anticorps contre CEA, d'un anticorps contre CA-19-9, d'une adhésine, qui est de préférence modifiée, et de combinaisons de ceux-ci.

6. Nanoparticule conjuguée selon l'une quelconque des revendications 1 à 5, dans laquelle la nanoparticule est sélectionnée parmi le groupe constitué de boîtes quantiques, agrégats de métal noble, nanoparticules d'oxyde de fer superparamagnétiques (IONP), micelles de copolymère séquencé, nanopiles, dendrimères, nanotubes, polymersomes, nanoparticules de XPclad®, nanoparticules constituées de silice amorphe entourée par une couche de phosphate de calcium luminescente cristalline, nanoparticules à coque de noyau de SiO₂/Zn₂SiO₄:Mn²⁺ et SiO₂/Ca₁₀(PO₄)₆OH:Eu³⁺ avec des diamètres en dessous de 100 nm et nanoparticules hybrides marquées par un colorant luminescent.

7. Nanoparticule conjuguée selon l'une quelconque des revendications 1 à 5, dans laquelle la nanoparticule a un noyau inorganique et une couche d'enrobage comprenant un composant d'imidazole, avec le diamètre le plus petit du noyau inorganique incluant la couche d'enrobage qui ne dépasse pas 15 nm.

8. Nanoparticule conjuguée selon la revendication 7, dans laquelle le composant d'imidazole est un peptide comprenant au moins un résidu histidyle, et de préférence un dipeptide.

9. Nanoparticule conjuguée selon la revendication 8, dans laquelle le composant d'imidazole est un mélange de différents dipeptides contenant un histidyle.

10. Nanoparticule conjuguée selon l'une quelconque des revendications 1 à 9, dans laquelle la nanoparticule est non inerte.

11. Nanoparticule conjuguée selon l'une quelconque des revendications 1 à 10 destinée à être utilisée dans le diagnostic in vivo ou le traitement d'une maladie sélectionnée parmi le groupe constitué de maladies auto-immunes telles que le diabète insulinodépendant, la sclérose en plaques, le syndrome de Sjögren ou le lupus érythémateux systémique (SLE) ; de maladies cutanées telles que le psoriasis ou la dermatite atopique ; de maladies inflammatoires chroniques telles que la polyarthrite rhumatoïde ou la maladie du côlon inflammatoire ; d'une lésion tissulaire ; de maladies allergiques et d'une maladie cancéreuse.

12. Nanoparticule conjuguée selon l'une quelconque des revendications 1 à 10 destinée à être utilisée in vivo pour distinguer un tissu anormal, pré-cancérogène ou cancérogène d'un tissu normal ou pour aider à l'évaluation visuelle au cours d'une intervention chirurgicale.

13. Nanoparticule selon la revendication 12, dans laquelle l'agent de contraste est capable d'identifier des cellules cancéreuses ou cellules de carcinome in situ.

14. Nanoparticule selon la revendication 13, dans laquelle les cellules cancéreuses devant être identifiées sont sélectionnées parmi la liste constituée de :
a) cellules d'adénocarcinome ;
b) cellules tumorales épithéliales thymiques ;
c) cellules de carcinome du col de l'utérus ;
d) cellules de lymphome non hodgkinien ;
e) cellules de carcinome du poumon ;
f) cellules de carcinome du pancréas ; et
g) cellules souches cancéreuses.

15. Utilisation de la nanoparticule conjuguée selon l'une quelconque des revendications 1 à 10 en tant qu'agent diagnostique ex vivo ou in vitro pour le diagnostic d'une maladie sélectionnée parmi le groupe constitué de maladies auto-immunes telles que le diabète insulinodépendant, la sclérose en plaques, le syndrome de Sjögren ou le lupus érythémateux systémique (SLE) ; de maladies cutanées telles que le psoriasis ou la dermatite atopique ; de maladies inflammatoires chroniques telles que la polyarthrite rhumatoïde ou la maladie du côlon inflammatoire ; d'une lésion tissulaire ; de maladies allergiques et d'une maladie cancéreuse.

16. Composition pharmaceutique comprenant la nanoparticule conjuguée selon l'une quelconque des revendications 1 à 10 et un excipient pharmaceutiquement acceptable.

17. Emballage à combinaison médicament/dosimètre comprenant :
a) un médicament devant être individuellement dosé, et
b) un système indicateur de diagnostic pour une propriété spécifique à un patient qui est pertinente pour l'action, l'effet secondaire, l'interaction, le métabolisme, l'absorption, la distribution, le métabolisme et l'élimination du médicament devant être administré à un patient,
dans lequel la propriété spécifique à un patient est sélectionnée parmi le groupe constitué d'une substance endogène, d'un mécanisme de régulation, d'un gène ou d'un système d'indication, et
dans lequel le médicament ou le système indicateur de diagnostic comprend une nanoparticule conjuguée selon l'une quelconque des revendications 1 à 10.
